(19) Europäisches Patentamt / European Patent Office / Office européen des brevets

(11) **EP 2 480 327 B1**

(12) **EUROPÄISCHE PATENTSCHRIFT**

(45) Veröffentlichungstag und Bekanntmachung des Hinweises auf die Patenterteilung:
**07.01.2015 Patentblatt 2015/02**

(21) Anmeldenummer: **10749814.9**

(22) Anmeldetag: **03.09.2010**

(51) Int Cl.:
*B01J 19/12* (2006.01)  *B01J 23/44* (2006.01)
*B01J 23/46* (2006.01)  *B01J 23/882* (2006.01)
*C07C 29/143* (2006.01)  *C07C 67/03* (2006.01)
*C10G 32/02* (2006.01)  *B01J 35/04* (2006.01)
*H05B 6/80* (2006.01)

(86) Internationale Anmeldenummer:
**PCT/EP2010/005427**

(87) Internationale Veröffentlichungsnummer:
**WO 2011/035852 (31.03.2011 Gazette 2011/13)**

(54) **VORRICHTUNG UND VERFAHREN ZUR KONTINUIERLICHEN DURCHFÜHRUNG HETEROGEN KATALYSIERTER CHEMISCHER REAKTIONEN BEI HOHEN TEMPERATUREN**

APPARATUS AND METHOD FOR CONTINUOUSLY CARRYING OUT HETEROGENEOUSLY CATALYZED CHEMICAL REACTIONS AT ELEVATED TEMPERATURES

DISPOSITIF ET PROCÉDÉ POUR LA MISE EN OEUVRE EN CONTINU DE RÉACTIONS CHIMIQUES À CATALYSE HÉTÉROGÈNE À DES TEMPÉRATURES ÉLEVÉES

(84) Benannte Vertragsstaaten:
**AT BE CH DE ES FR GB LI NL SE**

(30) Priorität: **22.09.2009 DE 102009042523**

(43) Veröffentlichungstag der Anmeldung:
**01.08.2012 Patentblatt 2012/31**

(73) Patentinhaber: **Clariant Finance (BVI) Limited Road Town, Tortola (VG)**

(72) Erfinder:
• **KRULL, Matthias**
  **55296 Harxheim (DE)**
• **MORSCHHÄUSER, Roman**
  **55122 Mainz (DE)**
• **SCHOLZ, Hans, Jürgen**
  **63755 Alzenau (DE)**

(74) Vertreter: **Mikulecky, Klaus et al Clariant Produkte (Deutschland) GmbH Patent & License Management Chemicals Industriepark Höchst, G 860 65926 Frankfurt am Main (DE)**

(56) Entgegenhaltungen:
**WO-A1-2009/121490   CN-A- 1 351 954**

• **GLASNOV TOMA N ET AL: "Microwave-assisted synthesis under continuous-flow conditions", MACROMOLECULAR: RAPID COMMUNICATIONS, WILEY VCH VERLAG, WEINHEIM, DE, Bd. 28, Nr. 4, 1. Januar 2007 (2007-01-01) , Seiten 395-410, XP002529633, ISSN: 1022-1336, DOI: DOI:10.1002/MARC. 200600665**

**Beschreibung**

Die vorliegende Erfindung betrifft eine Vorrichtung zur kontinuierlichen Durchführung heterogen katalysierter chemischer Reaktionen bei hohen Temperaturen und Überdruck unter Mikrowellenbestrahlung im technischen Maßstab.

Chemische Reaktionen lassen sich durch Katalysatoren beschleunigen. Besonders interessant sind dabei heterogen katalysierte Reaktionen, bei denen das Reaktionsgut an einem immobilisierten, katalytisch aktiven Material vorbeiströmt. Dabei verbleiben im Gegensatz zu homogen katalysierten Reaktionen im Wesentlichen keine Rückstände im so hergestellten Produkt und bei entsprechender Auslegung der Apparatur können große Produktmengen in hohen Raum-Zeit-Ausbeuten hergestellt werden. Besonders effizient sind katalysierte Reaktionen dann, wenn sie bei hohen Temperaturen durchgeführt werden, wobei die für die Durchführung dieser Reaktionen erforderlichen Temperaturen dann immer noch niedriger sind als ohne Anwesenheit des Katalysators.

Üblicherweise werden kontinuierliche, heterogen katalysierte Reaktionen in Reaktoren durchgeführt, die katalytisch aktive Festkörper bzw. mit katalytisch aktiven Spezies imprägnierte Trägermaterialien enthalten und vom Reaktionsgemisch durchströmt werden. Problematisch für die Durchführung heterogen katalysierter Reaktionen im industriellen Maßstab ist es oftmals, die für die Aktivierung der Reaktionen erforderlichen Temperaturen im Reaktionsgut homogen über den gesamten Querschnitt des Reaktors einzustellen. Die zur Gewährleistung des erforderlichen Wärmeübergangs benötigten höheren Mantel- oder auch Heizelementtemperaturen führen an den Heizflächen häufig zu lokalen (partiellen) Überhitzungen des Reaktionsguts und dadurch verursacht vielfach zu unerwünschten Nebenreaktionen oder auch Zersetzungen des Reaktionsgemischs, was Produktqualität und/oder Ausbeute mindert. Technisch sind der Temperaturerhöhung zudem durch die dann auftretenden Drücke Grenzen gesetzt. Zumindest in großen Reaktionsgefäßen von mehreren Litern oder mehreren Kubikmetern ist die Durchführung von Reaktionen unter hohen Drücken auf Grund der dann auftretenden Sicherheitsrisiken, wenn überhaupt, nur mit hohem technischem Aufwand realisierbar. Weiterhin führen die als Katalysator üblicherweise eingesetzten geschütteten Festkörperpackungen auf Grund ihrer geringen Porosität zu einem hohen Druckverlust im Reaktor.

Um kontinuierliche Reaktionen im industriellen Maßstab bei hohen Temperaturen und hohen Drücken durchzuführen, werden üblicherweise im Durchfluss betriebene dickwandige Behälter oder lange Rohre mit geringem Querschnitt (Strömungsrohre) eingesetzt, um einerseits den sicherheitstechnischen Anforderungen gerecht zu werden und andererseits die zur Erzielung interessanter Ausbeuten erforderlichen Verweilzeiten sicherzustellen. Für derartige Verfahren sind zum Einstellen der erforderlichen hohen Heizraten mit dem Maßstab steigende Manteltemperaturen erforderlich, die, wie bereits ausgeführt, zu unerwünschten Nebenreaktionen oder auch Zersetzungen führen können. Moderate Manteltemperaturen dagegen erfordern zur Erreichung der Zieltemperatur lange Verweilzeiten in Kesseln, wobei der Umsatz durch die dann auftretende Rückvermischung begrenzt wird; in Strömungsrohren erfordern geringe Temperaturgradienten entweder niedrige Strömungsgeschwindigkeiten und/oder lange Rohre. Während dieses langsamen Aufheizens werden bei vielen Reaktionen jedoch ebenfalls unerwünschte, zum Beispiel kinetisch begünstigte Nebenreaktionen beobachtet.

Ein neuerer Ansatz zur chemischen Synthese ist die Durchführung von Reaktionen im Mikrowellenfeld. So referiert M. Hájek (in A. Loupy, Microwaves in Organic Synthesis, Wiley 2006, Kapitel 13) eine Vielzahl von heterogen katalysierten Reaktionen, deren Reaktionsgeschwindigkeit und/oder Selektivität durch das Heizen mit Mikrowellen gesteigert wird. Diese Reaktionstechnik wird bisher hauptsächlich im Labormaßstab und nur selten im Kleintechnikumsmaßstab angewendet, da bisher keine Vorrichtungen bekannt sind, die die Herstellung von mehr als wenige Kilogramm pro Tag und somit eine Synthese im industriellen Maßstab ermöglichen.

Wolf et al. (AOSTRA J. of Research (1986) 3, 53) offenbaren Durchflussapparaturen, in denen mit Nickelpulver bzw. Nickel enthaltenden Legierungen katalysierte Reaktionen unter Mikrowellenbestrahlung durchgeführt werden. Das Design der Apparaturen ist für Umsetzungen im Labormaßstab ausgelegt und lässt sich zumindest auf Grund der begrenzten Eindringtiefe von Mikrowellen in Materie nicht auf einen für industrielle Anwendungen interessanten Maßstab vergrößern.

Shore et al. (Angew. Chem. 2006, 118, 2827-2832) offenbaren mit Palladium-Filmen beschichtete Kapillaren mit einem Durchmesser von 1.150 $\mu$m, in denen mikrowellenunterstützte Reaktionen durchgeführt werden. Durch die Palladium-Beschichtung werden deutlich erhöhte Umsätze erzielt.

WO 2009/064501 offenbart Verfahren zur katalytischen Entschwefelung von Rohölen unter Mikrowellenbestrahlung. Das vorgeschlagene Apparatedesign ist jedoch zumindest wegen der auf wenige Zentimeter begrenzten Eindringtiefe von Mikrowellen in Materie nicht auf einen industriellen Maßstab übertragbar.

WO 2006/024167 offenbart einen Mikroreaktor, in dem das durch eine mit katalytisch aktiven Substanzen beschichtete oder katalytisch aktive Körper enthaltende Kapillare strömende Reaktionsgut Mikrowellenstrahlung ausgesetzt wird, die senkrecht zur Längsachse der Kapillare eingestrahlt wird. Auf Grund der kurzen Bestrahlungszone sowie der nur wenige Zentimeter betragenden Eindringtiefe von Mikrowellen in das Reaktionsgut sind derartige Apparaturen auf Arbeiten im Labormaßstab beschränkt.

Mazzocchia et al. (C. R. Chemie, 2004, 7, 601-605) offenbaren mikrowellenunterstützte Umesterungen von Triglyceriden mit Methanol unter heterogener Katalyse durch Zeolithe. Dabei werden bei 170 °C und zweistündiger Bestrahlung im geschlossenen Gefäß nur moderate Umsätze erzielt.

Esveld et al. (Chem. Eng. Technol. 23 (2000), 429-435 offenbaren ein kontinuierliches Verfahren zur Herstellung von Wachsestern, bei dem Fettalkohol und Fettsäure lösemittelfrei in Gegenwart von Montmorillonit verestert werden. Auf einem Förderband wird das Reaktionsgemisch durch Mikrowellenstrahlung binnen 5 Minuten auf Reaktionstemperatur erhitzt und anschließend weitere 30 Minuten zur weitgehenden Entfernung des entstehenden Reaktionswassers bei dieser Temperatur gehalten. Dieses Verfahren ist naturgemäß nur auf hoch siedende Reaktanden (und Reaktionsprodukte) anwendbar.

CN 1351954 offenbart die Bestrahlung eines Katalytisch aktive Spezies tragenden Schaums in einem Rohr in einer Monomodalen Mikrowellenkavität.

Aufgabe der Erfindung war demzufolge die Bereitstellung einer Vorrichtung zur kontinuierlichen Durchführung heterogen katalysierter chemischer Reaktionen im technischen Maßstab bei hohen Temperaturen, in der das Reaktionsgut möglichst schnell und ohne partielle Überhitzung auf die gewünschte Reaktionstemperatur erhitzt werden kann. Weiterhin soll die Vorrichtung ein Arbeiten oberhalb des Atmosphärendrucks erlauben, so dass das Reaktionsgemisch auch bei erhöhten Temperaturen im flüssigen bzw. gelösten Zustand verbleibt. Die Vorrichtung soll eine hohe Raum-Zeit-Ausbeute, eine hohe energetische Effizienz und darüber hinaus ein sicheres und reproduzierbares Arbeiten ermöglichen. Eine weitere Aufgabe der Erfindung war die Bereitstellung einer Vorrichtung zur kontinuierlichen Durchführung von mikrowellenunterstützten Reaktionen, in der inhomogene Reaktionsgemische und/oder nicht bzw. nicht vollständig mischbare Reaktanden unter intensiver Durchmischung der Mikrowellenbestrahlung ausgesetzt werden können.

Überraschenderweise wurde gefunden, dass heterogen katalysierte chemische Reaktionen in einem kontinuierlichen Verfahren in technisch relevanten Mengen in einer Vorrichtung durchgeführt werden können, in der das umzusetzende Reaktionsgut durch Bestrahlung mit Mikrowellen in einem mikrowellentransparenten Rohr, dessen Längsachse sich in der Ausbreitungsrichtung der Mikrowellen eines Monomode-Mikrowellenapplikators befindet, wobei das mikrowellentransparente Rohr mit einem offenzelligen Schaum gefüllt ist, der katalytisch aktiv ist und/oder katalytisch aktive Spezies trägt, erhitzt wird. In der erfindungsgemäßen Vorrichtung werden der katalytisch aktive und/oder katalytisch aktive Spezies tragende Schaum und das ihn durchströmende Reaktionsgut homogen erhitzt und es kommt zu keinem wesentlichen Temperaturgradienten zwischen dem Inneren des offenporigen, katalytisch aktiven und/oder katalytisch aktive Spezies tragenden Schaums und der es umgebenden Rohrwand. Diese Vorrichtung erlaubt eine sehr schnelle aber trotzdem schonende Erhitzung des Reaktionsgutes mit sehr hohen Raum-Zeit-Ausbeuten und hoher energetischer Effizienz. Weiterhin können die Geschwindigkeit der Aufheizung und die Maximaltemperatur einfach, schnell und reproduzierbar durch Regelung der eingestrahlten Mikrowellenleistung eingestellt werden. Darüber hinaus findet eine sehr intensive Durchmischung des Reaktionsgemischs während der Mikrowellenbestrahlung statt.

Gegenstand der Erfindung ist eine Vorrichtung zur kontinuierlichen Durchführung heterogen katalysierter chemischer Reaktionen, umfassend ein mikrowellentransparentes Rohr, dessen Längsachse sich in der Ausbreitungsrichtung der Mikrowellen eines Monomode-Mikrowellenapplikators befindet, wobei das mikrowellentransparente Rohr mit einem offenzelligen, katalytisch aktive Spezies tragenden oder aus diesem bestehenden Schaum gefüllt ist.

Ein weiterer Gegenstand der Erfindung ist ein Verfahren zur kontinuierlichen Durchführung heterogen katalysierter chemischer Reaktionen, bei dem das Reaktionsgut durch Bestrahlung mit Mikrowellen in einem mikrowellentransparenten Rohr, dessen Längsachse sich in der Ausbreitungsrichtung der Mikrowellen eines Monomode-Mikrowellenapplikators befindet, umgesetzt wird, wobei das mikrowellentransparente Rohr mit einem offenzelligen, katalytisch aktive Spezies tragenden oder aus diesem bestehenden Schaum gefüllt ist.

Erfindungsgemäße Vorrichtung und erfindungsgemäßes Verfahren sind bevorzugt geeignet für Reaktionen der organischen Chemie. Insbesondere geeignet sind sie für solche Reaktionen, deren Reaktionsgeschwindigkeit sich durch die Anwesenheit katalytisch aktiver Spezies beschleunigen lässt. Unter Beschleunigung der Reaktionsgeschwindigkeit wird hier verstanden, dass bei ansonsten gleichen Reaktionsbedingungen wie beispielsweise Temperatur und Druck pro Zeiteinheit mehr Umsatz erfolgt als ohne Anwesenheit des Katalysators und/oder dass durch die Anwesenheit des Katalysators bei gleichem Umsatz ein erhöhter Durchsatz möglich ist. Weiterhin bevorzugt sind sie für Reaktionen, die ohne wesentliche exotherme Wärmetönung ablaufen. So sind erfindungsgemäße Vorrichtung und das darin durchgeführte Verfahren insbesondere für die Durchführung von Reaktionen geeignet, deren Wärmetönung $\Delta H$ kleiner als -50 kJ/mol und speziell kleiner als -20 kJ/mol wie beispielsweise kleiner als -10 kJ/mol ist. Besonders bevorzugt sind sie für endotherme Reaktionen, deren Wärmetönung $\Delta H$ größer als +0,1 kJ/mol und speziell zwischen +1 kJ/mol und +250 kJ/mol wie beispielsweise zwischen +2 kJ/mol und +100 kJ/mol ist. Da die katalytisch aktiven Spezies üblicherweise eine sehr hohe Mikrowellenabsorption zeigen, können gemäß dem erfindungsgemäßen Verfahren auch Substanzen bzw. Reaktionsgemische zur Reaktion gebracht werden, die selbst nur eine sehr geringe Mikrowellenabsorption zeigen. Beispiele für geeignete chemische Reaktionen sind Veresterungen, Umesterungen, Amidierungen, Esterspaltungen, Veretherungen, Acetalisierungen, En-Reaktionen, Diels-Alder-Reaktionen, Oxidationen, Ammoxidationen, Cyanierungen, Reduktionen, Hydrierungen, Hydrodeoxygenierungen, reduktive Aminierungen, nukleophile Substitutionen, Additionen, Hydrolysen, Isomerisierungen, Umlagerungen, Kondensationen, Decarboxylierungen, Eliminierungen, Cyclisierungen, Metathese-Reaktionen, metallkatalysierte Kupplungsreaktionen wie beispielsweise Suzuki-Reaktionen, Heck-Kupplungen, Sonogashira-Kupplungen, Stille-Kupplungen, Ullman-Reaktionen und Kumada-Kupplungen, Alkylierungen, Acylierungen wie beispielsweise Friedel-Crafts-Acylierungen, Crackreaktionen und Polymerisationen wie beispielsweise Polykondensationen. Auch für die hydrierende Entschwefelung von Rohölen und Mineralöldestillaten sind erfindungsgemäße Vorrichtung und Verfahren sehr gut geeignet. Neben den eigentlichen Reaktanden können die Reaktionsgemische auch Hilfsstoffe wie beispielsweise Lösemittel enthalten.

Unter offenzelligen Schäumen werden erfindungsgemäß Materialien mit zelliger Struktur und niedriger Dichte verstanden, bei denen die Zellwände im Wesentlichen nicht geschlossen sind. Die Strukturen bestehen somit bevorzugt aus miteinander verbundenen Stegen. Stege und Hohlräume bilden zwei kontinuierliche Netzwerke, die sich gegenseitig durchdringen. Der offenporige Schaum enthält folglich eine Vielzahl von Fließwegen innerhalb einer festen Struktur mit katalytisch aktiver bzw. katalytisch aktive Spezies enthaltender Oberfläche.

Bevorzugt ist der offenzellige Schaum so strukturiert, dass er einen Fluss des Reaktionsgutes in Längswie auch Querrichtung des mikrowellentransparenten Rohres, im Folgenden auch als Reaktionsrohr bezeichnet, möglich macht. Weiterhin ist er so strukturiert, dass er auch bei hohen Durchflussraten einen möglichst geringen Druckverlust im Reaktionsrohr verursacht. Der vom offenzelligen Schaum bedingte Druckverlust beträgt bevorzugt weniger als $5 \cdot 10^5$ Pa/m, besonders bevorzugt weniger als $2 \cdot 10^5$ Pa/m und insbesondere weniger als $1 \cdot 10^5$ Pa/m wie beispielsweise weniger als $0,5 \cdot 10^5$ Pa/m gegen Luft bei einer Strömungsgeschwindigkeit von 5 m/s. Die Porosität bevorzugter offenzelliger Schäume beträgt mindestens 20 %, bevorzugt 35 bis 99 %, besonders bevorzugt 50 bis 95 % wie beispielsweise 70 bis 90 %. Unter Porosität des Schaums wird dabei das Verhältnis aus Dichte des offenzelligen Schaums (ρ) zur Dichte des die Stege bildenden Materials ($\rho_S$) verstanden:

$$\text{Porosität} = (1 - \rho/\rho_S) \cdot 100$$

Bevorzugt beträgt die offene Porosität dabei mindestens 50 %, besonders bevorzugt 65 bis 100 % und insbesondere 70 bis 99 % wie beispielsweise 75 bis 95 % der insgesamt vorhandenen Zellen. Die in Anlehnung an ASTM D3576 bestimmbare Porenzahl bzw. Zellweite kann dabei in weiten Grenzen variieren. Sie liegt bevorzugt bei 1 bis 150 ppi (ppi = pores per inch) (0,39 - 59,06 ppcm), besonders bevorzugt zwischen 5 und 100 ppi (1,97 - 39,37 ppcm) und insbesondere zwischen 10 und 80 (3,94 - 31,5 ppcm) wie beispielsweise zwischen 20 und 60 ppi (7,87 - 23,62 ppcm). Die Porenweite bevorzugter offenzelliger Schäume liegt zwischen 1 cm und 0,01 mm und besonders bevorzugt zwischen 0,5 cm und 0,1 mm. Erfindungsgemäß geeignete offenzellige Schäume besitzen eine Festigkeit, die mindestens ausreicht, um dem Druck des sie durchströmenden Reaktionsguts zu widerstehen. Bevorzugte offenzellige Schäume haben eine über einen Stempeleindruck bestimmbare durchschnittliche Festigkeit (Bruchlast) von mindestens 100 N und insbesondere von 200 bis 15.000N wie beispielsweise von 300 bis 10.000 N. Die Festigkeit von Schaumkeramiken kann mit Hilfe eines einfachen Prüfverfahrens ermittelt werden, bei dem ein Prüfstempel mit einem definierten Durchmesser in die Schaumkeramik eingedrückt und die Kraft-Weg-Kurve aufgezeichnet wird. Die zur Zerstörung der Struktur benötigte Kraft wird als Maß für die Festigkeit angenommen. Sie wird als Bruchlast in der Einheit N angegeben und als Mittelwert aus einem Los von zehn Proben ermittelt. So kann die Festigkeit offenzelliger Schäume mit einer Prüfmaschine ermittelt werden, die einen Prüfstempel mit einem Durchmesser von 20 mm verwendet, wobei der Prüfkörper quer zur Bruchlast mindestens $40 \times 40$ mm messen und mindestens 10 mm dick sein soll. Als Bruchlast wird der erste Peak der Kraft-Weg-Kurve verwendet. Dieser ist bedingt durch das Einbrechen der obersten Stegebene.

In einer weiteren bevorzugten Ausführungsform ist der offenzelllige Schaum so ausgeformt, dass das Reaktionsgut in eine turbulente Strömung versetzt wird.

In einer bevorzugten Ausführungsform ist das mikrowellentransparente Rohr mit einem offenzelligen Schaum mit speziell geformter katalytisch aktiver Oberfläche gefüllt oder enthält aus einem solchen offenzelligen Schaum gebildete Füllkörper. In einer besonders bevorzugten Ausführungsform stellen die offenzelligen Schäume eine Oberfläche zur Beschichtung mit oder zur Einlagerung von katalytisch aktiven Spezies dar.

Bevorzugt ist der offenzellige Schaum so dimensioniert, dass er den Hohlraum des Reaktionsrohres zumindest in dem der Mikrowellenstrahlung ausgesetzten Bereich (Heizzone) im Wesentlichen ganz füllt. Besonders bevorzugt hat der Schaum dabei eine dem Innenraum des Reaktionsrohres entsprechende Geometrie und wird als Kern in diesem fixiert. Der Durchmesser derartiger Schaumkerne beträgt bevorzugt mindestens 90 %, besonders bevorzugt 95 bis 99,9 % wie beispielsweise 96 bis 99 % des inneren Durchmessers des Reaktionsrohres. In einer besonders bevorzugten Ausführungsform schließt der Schaumkern bündig mit der Wandung des Reaktionsrohres. Dadurch wird die Bildung von Kanälen vermieden, in denen das Reaktionsgemisch das Reaktionsrohr ohne Passieren des offenzellligen, katalytisch aktiven und/oder katalytisch aktive Spezies tragenden Schaums durchströmen kann. In einer speziellen Ausführungsform sind Reaktionsrohr und Schaum integral miteinander verbunden.

Der offenzellige Schaum kann in einer weiteren Ausführungsform auch in Form von katalytisch aktiven und/oder mit katalytisch aktiven Spezies imprägnierten Formkörpern in das Reaktionsrohr eingebracht werden. Als Formkörper eignen sich beliebige Formen, bevorzugt sind Tabletten, Ringe, Zylinder, Sterne, Wagenräder oder Kugeln, besonders bevorzugt sind Zylinder mit einem dem Querschnitt des Reaktionsrohres entsprechenden Durchmesser. Bevorzugt wird das Volumen des Reaktionsrohres durch die Schüttung der Formköper möglichst weitgehend gefüllt. Bevorzugt sind bei dieser Ausführungsform mehr als 50 Vol.-%, besonders bevorzugt 70 bis 100 Vol.-%, insbesondere 80 bis 99 Vol.-% wie beispielsweise 85 bis 95 Vol.-% des Reaktionsrohres mit Formkörpern (inklusive des Porenvolumens) gefüllt. Die Füllkörper werden bevorzugt durch Siebe, Fritten oder Querschittsverengungen im Reaktionsrohr gehalten.

Bevorzugt besitzen die für die Herstellung offenporiger Schäume eingesetzten Materialien Schmelzpunkte oberhalb der angestrebten Reaktionstemperatur, besonders bevorzugt mindestens 50 °C oberhalb der angestrebten Reaktionstemperatur, speziell mindestens 100 °C und insbesondere mindestens 200 °C oberhalb der angestrebten Reaktionstemperatur liegen.

In einer ersten bevorzugten Ausführungsform i) ist der offenzellige Schaum aus keramischem Material geformt. Auf viele Reaktionen haben keramische Materialien eine ausgeprägte katalytische Wirkung. Für den Einsatz in Reaktionen, bei denen das keramische Material selbst jedoch keine oder nur eine sehr geringe katalytische Aktivität zeigt, kann die Keramik in einer zweiten bevorzugten Ausführungsform ii) mit einer oder mehreren weiteren katalytisch aktiven Spezies durchsetzt oder imprägniert werden. In einer dritten bevorzugten Ausführungsform iii) ist der offenzellige Schaum im Wesentlichen aus einem oder mehreren Metallen, insbesondere Übergangsmetallen, Übergangsmetalle enthaltenden Legierungen, deren Oxiden oder deren Mischungen geformt.

Bevorzugte keramische Schäume der ersten Ausführungsform i) können aus gleichem oder unterschiedlichem Material wie das Reaktionsrohr bestehen. In einer bevorzugten Ausführungsform i) besteht der Schaum aus dem gleichen Material wie das Reaktionsrohr. Bevorzugte Materialien für erfindungsgemäß geeignete Keramiken sind beispielsweise Aluminiumoxid, Saphir, Zirkonoxid, Siliziumnitrid und ähnliches sowie deren Gemische. Auch Siliziumdioxid, Silikate und insbesondere Quarz sowie deren Mischungen mit den vorab genannten Keramiken sind geeignet.

Offenporige Keramikschäume sind im Stand der Technik bekannt. Derartige Keramikschäume werden in der Regel hergestellt, indem zunächst eine abbrennbare Schaumstruktur, üblicherweise eine organische Schaumstruktur wie beispielsweise ein Polyurethanschaum, mit einer Bestandteile zur Ausbildung einer Keramik enthaltenden, üblicherweise wässrigen Suspension (Schlicker) imprägniert wird. Danach wird die imprägnierte Schaumstruktur gegebenenfalls von überschüssiger Suspension befreit, zur Entfernung von Lösemittel getrocknet und anschließend bei einer Temperatur kalziniert, bei welcher die Schaumstruktur verbrennt und die auf der Schaumstruktur abgeschiedenen Bestandteile der Suspension zu einer Keramik zusammensintern. Dabei wird ein so genannter positiver Abdruck der Schaumstruktur mit der gleichen Makrostruktur, wie sie der ursprüngliche Polymerschaum besaß, erhalten. Insbesondere zur Erzielung mechanisch besonders stabiler offenporiger Schäume hat es sich bewährt, die Hohlräume eines polymeren Schaums mit zur Ausbildung einer Keramik enthaltenden Suspension zu füllen und anschließend durch pyrolytische Entfernung des originalen Polymers einen so genannten negativen Abdruck der Polymerstruktur zu erhalten. Durch Variation beispielsweise des polymeren Substrats, der Beschichtungsdichte des Substrats, der chemischen Zusammensetzung der Suspension und/oder der angewendeten Verfahrensschritte kann eine Vielzahl an verschiedenen offenzelligen Strukturen erzeugt werden. So können beispielsweise Schäume mit verschiedenen Geometrien, physikalischen Eigenschaften, Porositäten, Strömungswiderständen, spezifischen Oberflächen, katalytischen Aktivitäten und/oder Lebensdauern hergestellt werden. So ist es beispielsweise durch geeignete Wahl von Polymersubstrat, der Zusammensetzung des Schlickers und der Sinterbedingungen möglich, den offenzelligen Schaum hinsichtlich Makro-, Meso- und/oder Mikroporosität so zu strukturieren, dass er eine möglichst große für das Reaktionsgut zugängliche Oberfläche im Verhältnis zum Eigenvolumen besitzt. Unter "Makroporen" werden Poren mit Durchmessern von größer als etwa 500 Angström (A), unter "Mesoporen" Poren mit Durchmessern von etwa 20 Angström bis etwa 500 Ångström und unter "Mikroporen" Poren mit Durchmessern von weniger als etwa 20 Ångström verstanden.

Ein weiteres Beispiel für eine Methode zur Herstellung erfindungsgemäß geeigneter offenzelliger Schäume ist die Heterokoagulation. Dabei werden in kolloiden Systemen durch elektrostatische Wechselwirkung beispielsweise von zur Ausbildung von Keramik geeigneten Nanoteilchen mit Polymerteilchen Strukturen erhalten, die nach Filtration und Kalzinieren offenzellige Schäume mit erfindungsgemäß geeigneten Eigenschaften ergeben. Auch hier können durch Variation von beispielsweise Größe, Form, Ladung, Ladungsdichte und chemischer Zusammensetzung von Nanoteilchen wie auch Polymerteilchen die Eigenschaften der erhaltenen offenzelligen Schäume in weiten Grenzen eingestellt werden. Diese Methode eignet sich insbesondere zur Herstellung offenzelliger keramischer Schäume mit großer Oberfläche, hoher katalytischer Aktivität und geringem Fließwiderstand.

Geeignete offenzellige Schäume umfassen auch aus Aggregaten von Kugeln, Mikrokugeln, Körnern, Nanoröhrchen und/oder Hohlfasern gebildete und durch Kalzinieren verfestigte Strukturen, die eine große Oberfläche und eine Vielzahl von Fließwegen besitzen. Auch durch direktes Aufschäumen von Suspensionen Keramik bildender Materialien bzw. von Übergangsmetallen, deren Oxiden, Silikaten, Salzen und/oder Komplexen und gegebenenfalls Kalzinieren der erhaltenen Strukturen können erfindungsgemäße offenzellige Schäume erhalten werden.

Für Reaktionen, bei denen die Keramik selbst keine oder eine zu geringe katalytische Aktivität zeigt hat es sich in einer zweiten bevorzugten Ausführungsform ii) bewährt, die offenzelligen Keramiken der ersten bevorzugten Ausführungsform i) mit einer oder mehreren weiteren katalytisch aktiven Spezies zu durchsetzen, zu beschichten oder zu imprägnieren. So besteht der offenporige Schaum in einer besonders bevorzugten Ausführungsform ii) aus keramischen Material, in das katalytisch aktive Spezies eingelagert sind und/oder das mit katalytisch aktiven Spezies beschichtet oder imprägniert ist.

Als katalytisch aktive Spezies sind dabei prinzipiell alle festen Verbindungen geeignet, die in der Lage sind, eine oder mehrere chemische Reaktionen zu beschleunigen ohne dabei selbst verbraucht zu werden. Erfindungsgemäß bevorzugte Katalysatoren zeigen eine ausgeprägte Absorption von Mikrowellenstrahlung.

So kann der offenzellige keramische Schaum beispielsweise mit mindestens einem sauer oder basisch reagierenden Material durchsetzt sein. Derartige Schäume sind prinzipiell nach den gleichen Verfahren zugänglich wie die oben für die Ausführungsform i) beschriebenen offenzelligen Keramikschäume, wobei der zur Ausbildung einer Keramik enthaltenden Suspension katalytisch aktive Spezies oder deren Vorläufer zugesetzt werden, die in die Keramik bildende Masse eingelagert werden. Beispiele für derartige, basisch reagierende Zusätze sind beispielsweise Salze und Oxide der Alkali- und Erdalkalimetalle wie CaO, MgO, $Na_2O$, $K_2O$, $Na_2CO_3$, $K_2CO_3$, $Cs_2CO_3$ und deren Mischungen. Auch Montmorillonit, Zeolith, Ton, Silikate, Aluminiumoxid in saurer oder basischer Form können dem Schlicker als katalytisch aktive Spezies zugesetzt werden. Auch Borcarbid $B_4C$ kann dem Schlicker zugesetzt werden. Des gleichen können beispielsweise nach den für die Ausführungsform i) beschriebenen Verfahren zugängliche offenzellige Keramkischäume mit diesen oder anderen sauer oder basisch reagierenden Materialien beschichtet oder imprägniert sein, beispielsweise durch Auftragen einer Lösung oder Suspension mindestens eines katalytisch aktiven Materials und/oder dessen Vorläufers auf den offenzelligen keramischen Schaum und anschließendes Trocknen und gegebenenfalls Kalzinieren des so behandelten Schaums.

Weiterhin kann der offenzellige keramische Schaum mit mindestens einem Übergangsmetall, dessen Oxid, Sulfid, Silikat, Salz und/oder Komplex, Übergangsmetall enthaltenden Legierungen und/oder deren Mischungen durchsetzt oder mit diesen beschichtet oder imprägniert sein. Besonders geeignete Übergangsmetalle sind die Elemente der Gruppen IVA bis VIIIA sowie IA und IIA des Periodensystems. Beispiele für erfindungsgemäß geeignete katalytisch aktive Spezies sind Übergangsmetalle sowie deren Oxide und Silikate. Beispiele für geeignete Metalle sind Palladium, Nickel, Kobalt, Platin, Rhodium, Eisen, Kupfer, Chrom, Zink, Ruthenium, Osmium, Iridium, Silber, Gold, Vanadium, Wolfram, Titan, Mangan, Molybdän, Zirkon sowie Aluminium und deren Mischungen. Auch Mischungen sowie Legierungen verschiedener Metalle wie beispielsweise verschiedener Übergangsmetalle oder von Übergangsmetallen mit Metallen der Hauptgruppen, wie beispielsweise Alkali- und Erdalkalimetallen, können erfindungsgemäß als Katalysator eingesetzt werden. Beispiele für derartige Mischungen sind Kupfer-Zink, Nickel-Molybdän, Kobalt-Nickel-Molybdän, Kobalt-Molybdän, Nickel-Wolfram, Nickel-Natrium und Nickel-Wofram-Titan. Beispiele für geeignete Salze sind Acetate, Halogenide, Carbonate, Nitrate, Phosphate und/oder Sulfate; Beispiele für Liganden erfindungsgemäß geeigneter Komplexverbindungen sind ein- und mehrzähnige, bevorzugt zwei -, drei- und mehrzähnige Liganden wie beispielsweise Triphenylphosphin (TPP), Dibenzylidenaceton, 1,2-Bis(dicyclohexylphosphino)ethan, Cyclopentadienyl-; 2,3-Bis(diphenylphosphino)-bicyclo[2.2.1]hept-5-en (NorPhos), Bicyclo[2.2.1]hepta-2,5-dien (= Norbornadien, NOR), 2,2'-Bis[(N,N-dimethylamino)(phenyl)methyl]-1,1'-bisdicyclohexylphosphino)ferrocen (MandyPhos), 3,5-Dioxa-4-phosphacyclohepta[2,1-a;3,4-a]dinapthalen-4-yl)dimethylamin (MonoPhos), 4,4'-Di-tert-butyl-4,4',5,5'-tetrahydro-3,3'-bis-3H-dinaphtho[2,1-c:1',2'-e]phosphepin (Binapin), Butoxycarbonyl-4-diphenylphosphino-2-diphenylphosphinomethyl-pyrrolidin (BPPM), (1 R,2R)-Bis[(2-methoxyphenyl)phenylphosphino]ethan (DIPAMP), O-Isopropyliden-2,3-dihydroxy-1,4-bis(diphenylphosphino)butan (DIOP), 1,4-Diazabicyclo[2.2.2]octan (DABCO), Ethylendiamintetraacetat (EDTA), Tetramethylethylendiamin (TEMEDA), Ethylendiamin (EN), Diethylentriamin (DIEN), Iminodiacetat (IDA), Diethylentetramin, Triaminotriethylamin, Nitrilotriacetat (NTA), Ethylendiaminotriacetat (TED), Diethylentriaminpentaacetat (DTPA), 1,4,7,10-Tetraazacyclododecan-1,4,7,10-tetraacetat (DOTA), Oxalat (OX), Citrat, Tartrat, Dimethylglyoxim (DMG), Tris(pyrazolyl)borat (Tp), 2,2'-Binaphtyldiphenyldiphosphin (BINAP), 8-Hydroxychinolin, 2,2'-Bipyridin (BPY), 1,10-Phenanthrolin (PHEN), Dimercaptobernsteinsäure, Nitrilotriessigsäure (NTA) und Liganden basierend auf dem Gerüst des Porphyrins.

Die Beschichtung bzw. Imprägnierung der offenporigen Keramikschäume kann beispielsweise durch Tränken der Keramik mit Lösungen oder Suspensionen von Übergangsmetallen, deren Salzen und/oder Komplexen erfolgen, wobei eine Schicht aus fein verteiltem Metall bzw. Metallsalz und/oder Metallkomplex aufgetragen wird. Die Imprägnierung erfolgt bevorzugt mit löslichen Salzen und/oder Komplexen oben genannter Metalle wie beispielsweise den Acetaten, Halogeniden, Nitraten, Carbonaten, Phosphaten und/oder Sulfaten. Neben Wasser sind polare organische Lösemittel sowie Mischungen aus Wasser und einem oder mehreren polaren organischen Lösemitteln zur Herstellung der Lösungen und/oder Suspensionen bevorzugt. Die Übergangsmetalle wie auch ihre Salze und Komplexe können beispielsweise durch Absorption und insbesondere Komplexe durch chemische Bindung auf dem offenzelligen Schaum fixiert werden. Sie können weiterhin durch beispielsweise Kalzinieren oder andere chemische Prozesse in die entsprechenden Oxide der Metalle überführt werden. In einer besonders bevorzugten Ausführungsform kann insbesondere der positive Abdruck eines Polymerschaums mit katalytisch aktiven Spezies beschichtet werden. Die erhaltenen Strukturen können anschließend zum Beispiel durch Sintern weiter optimiert werden. Das Beschichten der offenzelligen Schäume mit katalytisch aktiven Spezies kann weiterhin beispielsweise durch Pulverbeschichtung, Galvanisieren oder Aufdampfen erfolgen.

Mit katalytisch aktiven Spezies durchsetzte und/oder beschichtete offenzellige Schäume enthalten bevorzugt 0,001 bis 25 Gew.-% und besonders bevorzugt 0,05 bis 20 Gew.-% wie beispielsweise 0,1 bis 10 Gew.-% an katalytisch aktiven Spezies bezogen auf das Gesamtgewicht des Schaums.

Die offenzelligen Schäume der dritten bevorzugten Ausführungsform iii) werden im Wesentlichen aus einem oder mehreren Übergangsmetallen, Übergangsmetalle enthaltenden Legierungen, deren Oxiden, Sulfiden, Silikaten oder deren Mischungen geformt. Bevorzugte katalytisch aktive Materialien sind Übergangsmetalle und deren Oxide. Besonders geeignete Übergangsmetalle sind die Elemente der Gruppen IVA bis VIIIA sowie IA und IIA des Periodensystems. Beispiele für geeignete Metalle sind Palladium, Nickel, Kobalt, Platin, Rhodium, Eisen, Kupfer, Chrom, Zink, Ruthenium, Osmium, Iridium, Silber, Gold, Vanadium, Wolfram, Titan, Mangan, Molybdän, Zirkon sowie Aluminium und deren Mischungen. Auch Mischungen sowie Legierungen verschiedener Metalle wie beispielsweise verschiedener Übergangsmetalle oder von Übergangsmetallen mit Metallen der Hauptgruppen wie beispielsweise Alkali- und Erdalkalimetallen können erfindungsgemäß als Katalysator eingesetzt werden. Beispiele für derartige Mischungen sind Kupfer-Zink, Nickel-Molybdän, Kobalt-Nickel-Molybdän, Kobalt-Molybdän, Nickel-Wolfram, Nickel-Natrium und Nickel-Wofram-Titan.

Die Herstellung derartiger Schäume kann in Analogie zu den für die Herstellung der keramischen Schäume der ersten Ausführungsform i) beschriebenen Verfahren erfolgen. Dabei wird beispielsweise ein Polymerschaum mit der Lösung oder Suspension eines oder mehrerer Übergangsmetalle, Übergangsmetalle enthaltenden Legierungen, deren Oxiden, Sulfiden, Salzen und/oder Komplexen imprägniert und nach Entfernen überschüssigen Materials und Trocknen das Polymer pyrolytisch entfernt. Die Imprägnierung des Polymerschaums erfolgt dabei bevorzugt mit löslichen Salzen und/oder Komplexen oben genannter Metalle wie beispielsweise den Acetaten, Halogeniden, Nitraten, Carbonaten, Phosphaten und/oder Sulfaten. Neben Wasser sind polare organische Lösemittel sowie Mischungen aus Wasser und einem oder mehreren polaren organischen Lösemitteln zur Herstellung der Lösungen und/oder Suspensionen bevorzugt. Dabei sind sowohl positive wie auch negative Abdrücke des Polymerschaums herstellbar. Es sind zahlreiche weitere Methoden zur Herstellung erfindungsgemäß geeigneter offenzelliger Schäume bekannt wie beispielsweise das direkte Aufschäumen katalytisch aktiver Metalle oder Metalloxide.

Oftmals hat es sich bewährt, den offenporigen, katalytisch aktiven oder katalytisch aktive Spezies tragenden Schaum vor seinem Einsatz zu aktivieren. Dies kann beispielsweise durch thermische Behandlung des Schaums erfolgen. Besonders bewährt hat sich dabei die thermische Behandlung unter Bestrahlung mit Mikrowellen. Metalle oder Metalloxide können vor ihrer Verwendung beispielsweise bei der hydrierenden Entschwefelung auf bekannte Weise in die Sulfide überführt werden.

Als Mikrowellen werden elektromagnetische Strahlen mit einer Wellenlänge zwischen etwa 1 cm und 1 m und Frequenzen zwischen etwa 300 MHz und 30 GHz bezeichnet. Dieser Frequenzbereich ist prinzipiell für das erfindungsgemäße Verfahren geeignet. Bevorzugt wird erfindungsgemäß Mikrowellenstrahlung mit den für industrielle, wissenschaftliche, medizinische; häusliche oder ähnliche Anwendungen freigegebenen Frequenzen verwendet wie beispielsweise Frequenzen von 915 MHz, 2,45 GHz, 5,8 GHz oder 24,12 GHz.

In einer bevorzugten Ausführungsform handelt es sich bei dem mikrowellentransparenten Rohr der erfindungsgemäßen Vorrichtung um ein druckfestes, chemisch inertes Rohr (Reaktionsrohr), bei dessen Durchströmen das Reaktionsgut Mikrowellenstrahlung ausgesetzt wird. Das Reaktionsrohr ist dabei im Wesentlichen gerade. Das Erhitzen des Reaktionsguts erfolgt bevorzugt in einem mikrowellentransparenten, geraden Rohr, dessen Längsachse sich in der Ausbreitungsrichtung der Mikrowellen eines Monomode-Mikrowellenapplikators befindet.

Bevorzugt erfolgt die Bestrahlung des Reaktionsgutes mit Mikrowellen in einem mikrowellentransparenten, geraden Reaktionsrohr, das sich innerhalb eines mit einem Mikrowellengenerator verbundenen Hohlleiters befindet. Bevorzugt fluchtet das Reaktionsrohr axial mit der zentralen Symmetrieachse des Hohlleiters.

Der als Mikrowellenapplikator fungierende Hohlleiter ist bevorzugt als Hohlraumresonator ausgeformt. Bevorzugt wird die Länge des Hohlraumresonators so dimensioniert, dass sich in ihm eine stehende Welle ausbildet. Weiterhin bevorzugt werden die vom Reaktionsgut im Hohlleiter nicht absorbierten Mikrowellen an seinem Ende reflektiert. Durch die Ausformung des Mikrowellenapplikators als Resonator vom Reflexionstyp werden eine lokale Erhöhung der elektrischen Feldstärke bei gleicher vom Generator zugeführter Leistung und eine erhöhte Energieausnutzung erzielt.

Der Hohlraumresonator wird bevorzugt im $E_{01n}$-Mode betrieben, wobei n für eine ganze Zahl steht und die Anzahl der Feldmaxima der Mikrowelle entlang der zentralen Symmetrieachse des Resonators angibt. Bei diesem Betrieb ist das elektrische Feld in Richtung der zentralen Symmetrieachse des Hohlraumresonators gerichtet. Es hat im Bereich der zentralen Symmetrieachse ein Maximum und nimmt zur Mantelfläche hin auf den Wert Null ab. Diese Feldkonfiguration liegt rotationssymmetrisch um die zentrale Symmetrieachse vor. Durch Verwendung eines Hohlraumresonators mit einer Länge, bei der n eine ganze Zahl ist, wird die Ausbildung einer stehenden Welle ermöglicht. Je nach der gewünschten Strömungsgeschwindigkeit des Reaktionsguts durch das Reaktionsrohr, der benötigten Temperatur und der benötigten Verweilzeit im Resonator wird die Länge des Resonators relativ zu der Wellenlänge der eingesetzten Mikrowellenstrahlung ausgewählt. Bevorzugt ist n eine ganze Zahl von 1 bis 200, besonders bevorzugt von 2 bis 100, insbesondere von 3 bis 50 speziell von 4 bis 20 wie beispielsweise drei, vier, fünf, sechs, sieben, acht, neun oder zehn.

Die $E_{01n}$-Mode des Hohlraumresonators wird in Englischer Sprache auch als $TM_{01n}$-Mode bezeichnet, siehe beispielsweise K. Lange, K.H. Löcherer, Taschenbuch der Hochfrequenztechnik", Band 2, Seite K21 ff.

Die Einstrahlung der Mikrowellenenergie in den als Mikrowellenapplikator fungierenden Hohlleiter kann über geeignet dimensionierte Löcher oder Schlitze erfolgen. In einer erfindungsgemäß besonders bevorzugten Ausführungsform erfolgt die Bestrahlung des Reaktionsguts mit Mikrowellen in einem Reaktionsrohr, das sich in einem Hohlleiter mit koaxialem Übergang der Mikrowellen befindet. Für dieses Verfahren besonders bevorzugte Mikrowelleneinrichtungen sind aus einem Hohlraumresonator, einer Koppeleinrichtung zum Einkoppeln eines Mikrowellenfeldes in den Hohlraumresonator und mit je einer Öffnung an zwei gegenüber liegenden Stirnwänden zum Hindurchführen des Reaktionsrohres durch den Resonator aufgebaut. Die Einkopplung der Mikrowellen in den Hohlraumresonator erfolgt bevorzugt über einen Koppelstift, der in den Hohlraumresonator hineinragt. Bevorzugt ist der Koppelstift als ein als Kopplungsantenne fungierendes, bevorzugt metallisches Innenleiterrohr ausgeformt. In einer besonders bevorzugten Ausführungsform ragt dieser Koppelstift durch eine der stirnseitigen Öffnungen in den Hohlraumresonator hinein. Besonders bevorzugt schließt sich das Reaktionsrohr an das Innenleiterrohr des koaxialen Übergangs an und speziell wird es durch dessen Hohlraum hindurch in den Hohlraumresonator geführt. Bevorzugt fluchtet das Reaktionsrohr axial mit einer zentralen Symmetrieachse des Hohlraumresonators. Dazu weist der Hohlraumresonator bevorzugt je eine zentrische Öffnung an zwei gegenüber liegenden Stirnwänden zum Hindurchführen des Reaktionsrohres auf.

Die Einspeisung der Mikrowellen in den Koppelstift bzw. in das als Kopplungsantenne fungierende Innenleiterrohr kann beispielsweise mittels einer koaxialen Anschlussleitung erfolgen. In einer bevorzugten Ausführungsform wird das Mikrowellenfeld über einen Hohlleiter dem Resonator zugeführt, wobei das aus dem Hohlraumresonator herausragende Ende des Koppelstifts in eine Öffnung, die sich in der Wand des Hohlleiters befindet, in den Hohlleiter hineingeführt ist und von dem Hohlleiter Mikrowellenenergie entnimmt und in den Resonator koppelt.

In einer speziellen Ausführungsform erfolgt die Bestrahlung des Reaktionsgutes mit Mikrowellen in einem mikrowellentransparenten Reaktionsrohr, das sich axialsymmetrisch in einem $E_{01n}$-Rundhohlleiter mit koaxialem Übergang der Mikrowellen befindet. Dabei wird das Reaktionsrohr durch den Hohlraum eines als Kopplungsantenne fungierenden Innenleiterrohres in den Hohlraumresonator geführt. In einer weiteren bevorzugten Ausführungsform erfolgt die Bestrahlung des Reaktionsgutes mit Mikrowellen in einem mikrowellentransparenten Reaktionsrohr, das durch einen $E_{01n}$-Hohlraumresonator mit axialer Einspeisung der Mikrowellen geführt wird, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das durch einen $E_{01n}$-Hohlraumresonator mit axialer Einspeisung der Mikrowellen geführt wird, wobei die Länge des Hohlraumresonators so bemessen ist, dass sich eine stehende Welle mit n = 2 oder mehr Feldmaxima der Mikrowelle ausbildet. In einer weiteren bevorzugten Ausführungsform erfolgt die Bestrahlung des Reaktionsgutes mit Mikrowellen in einem mikrowellentransparenten Reaktionsrohr, das sich axialsymmetrisch in einem kreiszylindrischen $E_{01n}$-Hohlraumresonator mit koaxialem Übergang der Mikrowellen befindet, wobei die Länge des Hohlraumresonators so bemessen ist, dass sich n = 2 oder mehr Feldmaxima der Mikrowelle ausbilden. In einer weiteren bevorzugten Ausführungsform erfolgt die Bestrahlung des Reaktionsgemisches mit Mikrowellen in einem mikrowellentransparenten Reaktionsrohr, das sich axialsymmetrisch in einem kreiszylindrischen $E_{01n}$-Hohlraumresonator mit koaxialem Übergang der Mikrowellen befindet, wobei die Länge des Hohlraumresonators so bemessen ist, dass sich eine stehende Welle mit n = 2 oder mehr Feldmaxima der Mikrowelle ausbildet.

Mikrowellengeneratoren, wie beispielsweise das Magnetron, das Klystron und das Gyrotron sind dem Fachmann bekannt.

Die zur Mikrowellenbestrahlung eingesetzten Reaktionsrohre sind bevorzugt aus mikrowellentransparentem, hoch schmelzendem Material gefertigt. Besonders bevorzugt werden nichtmetallische Reaktionsrohre eingesetzt. Unter mikrowellentransparent werden hier Werkstoffe verstanden, die selbst möglichst wenig Mikrowellenenergie absorbieren und in Wärme umwandeln. Als Maß für die Fähigkeit eines Stoffes, Mikrowellenenergie zu absorbieren und in Wärme zu überführen wird oftmals der dielektrische Verlustfaktor tan $\delta = \varepsilon''/\varepsilon'$ herangezogen. Der dielektrische Verlustfaktor tan $\delta$ ist definiert als das Verhältnis aus dielektrischem Verlust $\varepsilon''$ und Dielektrizitätskonstante $\varepsilon'$. Beispiele für tan $\delta$-Werte verschiedener Materialien sind beispielsweise in D. Bogdal, Microwave-assisted Organic Synthesis, Elsevier 2005 wiedergegeben. Für erfindungsgemäß geeignete Reaktionsrohre werden Materialen mit bei 2,45 GHz und 25 °C gemessenen tan $\delta$-Werten von unter 0,01, insbesondere unter 0,005 und speziell unter 0,001 bevorzugt. Als bevorzugte mikrowellentransparente und temperaturstabile Materialien kommen in erster Linie Werkstoffe auf mineralischer Basis wie beispielsweise Quarz, Aluminiumoxid, Saphir, Zirkonoxid, Siliziumnitrid und ähnliches in Betracht. Auch temperaturstabile Kunststoffe wie insbesondere Fluorpolymere wie beispielsweise Teflon, und technische Kunststoffe wie Polypropylen, oder Polyaryletherketone wie beispielsweise glasfaserverstärktes Polyetheretherketon (PEEK) sind als Rohrmaterialien geeignet. Um den Temperaturbedingungen während der Reaktion zu widerstehen haben sich insbesondere mit diesen Kunststoffen beschichtete Mineralien wie Quarz oder Aluminiumoxid als Reaktormaterialien bewährt.

Erfindungsgemäß besonders für die Mikrowellenbestrahlung geeignete Reaktionsrohre haben einen Innendurchmesser von einem Millimeter bis ca. 50 cm, insbesondere zwischen 2 mm und 35 cm und speziell zwischen 5 mm und 15 cm wie beispielsweise zwischen 10 mm und 7 cm. Unter Reaktionsrohren werden hier Gefäße verstanden, deren Verhältnis von Länge zu Durchmesser größer als 5, bevorzugt zwischen 10 und 100.000, besonders bevorzugt zwischen 20 und 10.000 wie beispielsweise zwischen 30 und 1.000 ist. Unter der Länge des Reaktionsrohres wird hier die Strecke des Rohres verstanden, auf der die Mikrowellenbestrahlung erfolgt.

Erfindungsgemäß besonders geeignete $E_{01}$-Hohlraumresonatoren haben bevorzugt einen Durchmesser, der mindestens der halben Wellenlänge der verwendeten Mikrowellenstrahlung entspricht. Bevorzugt beträgt der Durchmesser des Hohlraumresonators das 1,0- bis 10-fache, besonders bevorzugt das 1,1- bis 5-fache und insbesondere das 2,1- bis 2,6-fache der halben Wellenlänge der verwendeten Mikrowellenstrahlung. Bevorzugt hat der $E_{01}$-Hohlraumresonator einen runden Querschnitt, was auch als $E_{01}$-Rundhohlleiter bezeichnet wird. Besonders bevorzugt hat er eine zylindrische Form und speziell eine kreiszylindrische Form.

Die Verweilzeit des Reaktionsgutes in dem der Mikrowellenbestrahlung ausgesetzten Bereich des Reaktionsrohres (Heizzone) hängt von verschiedenen Faktoren wie beispielsweise der Geometrie des Reaktionsrohres, der eingestrahlten Mikrowellenenergie, der spezifischen Mikrowellenabsorption des Reaktionsgutes und der gewünschten Reaktionstemperatur ab. Die Verweilzeit des Reaktionsgutes im Reaktionsrohr und speziell in der Heizzone beträgt üblicherweise weniger als 30 Minuten, bevorzugt zwischen 0,01 Sekunden und 15 Minuten, besonders bevorzugt zwischen 0,1 Sekunden und 10 Minuten und insbesondere zwischen einer Sekunde und 5 Minuten wie beispielsweise zwischen 5 Sekunden und 2 Minuten. Die Intensität (Leistung) der Mikrowellenstrahlung wird dabei so eingestellt, dass das Reaktionsgut beim Verlassen des Reaktionsrohres die gewünschte Reaktionstemperatur besitzt.

Die für die Durchführung des erfindungsgemäßen Verfahrens in den Hohlraumresonator einzustrahlende Mikrowellenleistung ist insbesondere abhängig von der angestrebten Reaktionstemperatur, aber auch von der Geometrie des Reaktionsrohres und damit des Reaktionsvolumens sowie der Durchflussgeschwindigkeit des Reaktionsgutes durch das Reaktionsrohr und der Dauer der für die Durchführung der chemischen Reaktion erforderlichen Bestrahlung. Die einzustrahlende Mikrowellenleistung liegt üblicherweise zwischen 200 W und mehreren 100 kW und insbesondere zwischen 500 W und 100 kW wie beispielsweise zwischen 1 kW und 70 kW. Sie kann über einen oder mehrere Mikrowellengeneratoren erzeugt werden. Zur Optimierung der Raum-Zeit-Ausbeute wird die Mikrowellenleistung bevorzugt so eingestellt, dass das Reaktionsgemisch in möglichst kurzer Zeit die gewünschte Reaktionstemperatur erreicht, ohne dass es jedoch zu elektrischen Entladungen im Mikrowellenapplikator kommt.

Bevorzugt wird der durch die Mikrowellenbestrahlung bedingte Temperaturanstieg beispielsweise durch Regelung der Mikrowellenintensität und/oder der Durchflussgeschwindigkeit auf maximal 500 °C begrenzt. Die Temperatur kann beispielsweise an der Oberfläche des Reaktionsrohres gemessen werden; bevorzugt wird sie am Reaktionsgut direkt nach dem Verlassen der Heizzone bestimmt. Besonders bewährt hat sich die Durchführung des erfindungsgemäßen Verfahrens bei Temperaturen zwischen 100 und 400 °C, insbesondere zwischen 120 und 350 °C und speziell zwischen 150 und 300 °C wie beispielsweise bei Temperaturen zwischen 180 und 270 °C. In einer bevorzugten Ausführungsform wird das Umsetzungsprodukt direkt nach Beendigung der Mikrowellenbestrahlung möglichst schnell auf Temperaturen unterhalb 120 °C, bevorzugt unterhalb 100 °C und speziell unterhalb 60 °C abgekühlt.

In der Reaktionszone können Edukt, Produkt, gegebenenfalls Nebenprodukt und, sofern anwesend, Lösemittel durch die Temperaturerhöhung zu einem Druckaufbau führen. Dieser Überdruck kann bei der Entspannung zur Verflüchtigung und Abtrennung von überschüssigen Edukt(en), Produkt, Nebenprodukt sowie gegebenenfalls Lösemittel und/oder zur Abkühlung des Reaktionsprodukts genutzt werden.

Die Umsetzung des Reaktionsguts ist beim Verlassen des Reaktionsrohres oftmals noch nicht im chemischen Gleichgewicht. In einer bevorzugten Ausführungsform wird das Reaktionsgemisch daher nach Passieren des Reaktionsrohres direkt, das heißt ohne Zwischenkühlung in eine isotherme Reaktionsstrecke überführt, in der es für eine gewisse Zeit auf Reaktionstemperatur gehalten wird. Bevorzugt ist auch die isotherme Reaktionsstrecke mit katalytisch aktivem bzw. katalytisch aktive Spezies enthaltendem offenporigem Schaum gefüllt. Erst nach Verlassen der Reaktionsstrecke wird das Reaktionsgemisch gegebenenfalls entspannt und abgekühlt. Unter der direkten Überführung aus dem Reaktionsrohr in die isothermen Reaktionsstrecke ist zu verstehen, dass zwischen Reaktionsrohr und Reaktionsstrecke keine aktiven Maßnahmen zum Zuführen und insbesondere zum Abführen von Wärme getroffen werden. Bevorzugt ist die Temperaturdifferenz zwischen Verlassen des Reaktionsrohres bis zum Eintritt in die Reaktionsstrecke kleiner als ± 30 °C, bevorzugt kleiner ± 20 °C, besonders bevorzugt kleiner ± 10 °C und insbesondere kleiner ± 5 °C. In einer speziellen Ausführungsform entspricht die Temperatur des Reaktionsguts beim Eintritt in die Reaktionsstrecke der Temperatur beim Verlassen des Reaktionsrohres. Diese Ausführungsvariante ermöglicht eine schnelle und gezielte Erhitzung des Reaktionsgutes auf die gewünschte Reaktionstemperatur ohne partielle Überhitzung und sodann ein Verweilen bei dieser Reaktionstemperatur für einen definierten Zeitraum bevor es abgekühlt wird. So können eine erhöhte Raum-Zeit-Ausbeute, eine erhöhte energetische Effizienz und darüber hinaus ein sicheres und reproduzierbares Arbeiten erzielt werden. In dieser Ausführungsform wird das Reaktionsgut bevorzugt direkt nach Verlassen der Reaktionsstrecke möglichst schnell auf Temperaturen unterhalb 120 °C, bevorzugt unterhalb 100 °C und speziell unterhalb 60 °C abgekühlt.

Als isotherme Reaktionsstrecke kommen alle chemisch inerten Gefäße in Betracht, die ein Verweilen der Reaktionsgemische bei der im Reaktionsrohr eingestellten Temperatur ermöglichen. Unter isothermer Reaktionsstrecke wird verstanden, dass die Temperatur des Reaktionsgutes in der Reaktionsstrecke gegenüber der Eintrittstemperatur auf ± 30 °C, bevorzugt auf ± 20 °C, besonders bevorzugt auf ± 10 °C und insbesondere auf ± 5 °C konstant gehalten wird. Somit hat das Reaktionsgut beim Verlassen der Reaktionsstrecke eine Temperatur, die maximal ± 30 °C, bevorzugt ± 20 °C, besonders bevorzugt ± 10 °C und insbesondere ± 5 °C von der Temperatur beim Eintritt in die Reaktionsstrecke abweicht.

Neben kontinuierlich betriebenen Rührbehältern und Behälterkaskaden sind insbesondere Rohre als isotherme Reaktionsstrecke geeignet. Diese Reaktionsstrecken können aus verschiedenen Materialien wie beispielsweise Metallen, Keramik, Glas, Quarz oder Kunststoffen bestehen mit der Maßgabe, dass diese unter den gewählten Temperatur- und Druckbedingungen mechanisch stabil und chemisch inert sind. Besonders bewährt haben sich dabei thermisch isolierte Gefäße. Die Verweilzeit des Reaktionsguts in der Reaktionsstrecke kann beispielsweise über das Volumen der Reaktionsstrecke eingestellt werden. Bei Verwendung von Rührbehältern und Behälterkaskaden hat es sich gleichermaßen bewährt, die Verweilzeit über den Füllgrad der Behälter einzustellen. In einer bevorzugten Ausführungsform ist auch die isotherme Reaktionsstrecke mit offenporigem, katalytisch aktivem bzw. katalytisch aktive Spezies enthaltendem Schaum beladen. Dabei kann es sich um den gleichen Schaum wie im Reaktionsrohr oder einen davon verschiedenen Schaum handeln.

In einer bevorzugten Ausführungsform wird als Reaktionsstrecke ein Rohr verwendet. Dabei kann es sich um eine Verlängerung des mikrowellentransparenten Reaktionsrohres im Anschluss an die Heizzone oder auch ein separates, mit dem Reaktionsrohr in Verbindung stehendes Rohr aus gleichem oder unterschiedlichem Material handeln. Über die Länge des Rohres und/oder seinen Querschnitt lässt sich bei gegebener Flussrate die Verweilzeit des Reaktionsgutes bestimmen. Das als Reaktionsstrecke fungierende Rohr ist im einfachsten Fall thermisch isoliert, so dass die beim Eintritt des Reaktionsgutes in die Reaktionsstrecke herrschende Temperatur in den oben gegebenen Grenzen gehalten wird. Dem Reaktionsgut kann in der Reaktionsstrecke aber auch beispielsweise mittels eines Wärmeträgers bzw. Kühlmediums gezielt Energie zu- oder abgeführt werden. Diese Ausführungsform hat sich insbesondere zum Anfahren der Vorrichtung bzw. des Verfahrens bewährt. So kann die Reaktionsstrecke beispielsweise als Rohrschlange oder als Rohrbündel ausgestaltet sein, die sich in einem Heiz- oder Kühlbad befindet oder in Form eines Doppelmantelrohres mit einem Heiz- oder Kühlmedium beaufschlagt werden. Die Reaktionsstrecke kann sich auch in einem weiteren Mikrowellenapplikator befinden, in dem das Reaktionsgut nochmals mit Mikrowellen behandelt wird. Dabei können sowohl im Monomode- wie auch Multimode arbeitende Applikatoren zum Einsatz kommen.

Die Verweilzeit des Reaktionsgutes in der isothermen Reaktionsstrecke ist abhängig von der Reaktionsgeschwindigkeit der durchgeführten Reaktion sowie der Geschwindigkeit eventueller unerwünschter Nebenreaktionen. Im Idealfall wird die Verweilzeit in der Reaktionsstrecke so bemessen, dass der durch die herrschenden Bedingungen definierte thermische Gleichgewichtszustand erreicht wird. Üblicherweise liegt die Verweilzeit zwischen 1 Sekunde und 10 Stunden, bevorzugt zwischen 10 Sekunden und 2 Stunden, besonders bevorzugt zwischen 20 Sekunden und 60 Minuten wie beispielsweise zwischen 30 Sekunden und 30 Minuten. Weiterhin bevorzugt liegt das Verhältnis zwischen Verweilzeit des Reaktionsgutes in der Reaktionsstrecke zur Verweilzeit in der Heizzone zwischen 1:2 und 100:1, besonders bevorzugt 1:1 bis 50:1 und insbesondere zwischen 1:1,5 und 10:1.

Die erfindungsgemäße Vorrichtung ist üblicherweise am Einlass mit einer Dosierpumpe sowie einem Manometer und am Auslass mit einer Druckhaltevorrichtung und einer Kühlvorrichtung wie beispielsweise einem Wärmetauscher versehen. Damit sind Reaktionen in einem sehr weiten Druck- und Temperaturbereich möglich.

Die Abkühlung des Reaktionsgemischs nach Verlassen des Reaktionsrohres bzw. der isothermen Reaktionsstrecke kann beispielsweise mittels Wärmetauscher oder adiabatischer Expansion oder Verdünnen mit kaltem Lösemittel erfolgen.

Üblicherweise erfolgt die Entspannung des Reaktionsgemischs auf Atmosphärendruck, sie kann aber für anschließende Verfahrensschritte bzw. bei Verwendung spezieller Apparaturen auch auf höhere oder niedrigere Drücke erfolgen. So hat es sich beispielsweise bewährt, das Reaktionsgemisch zur Abtrennung von Lösemittel und/oder unumgesetzten Edukten auf Drücke deutlich unterhalb des Atmosphärendrucks zu entspannen. Die Abkühlung kann in Abhängigkeit von den Eigenschaften der umgesetzten Produkte und der vorgesehenen weiteren Verfahrensschritte vor oder auch nach Druckabsenkung oder bei einem dazwischen liegenden Druck erfolgen.

Die Herstellung der Reaktionsgemische kann kontinuierlich, diskontinuierlich oder auch in semi-Batch-Prozessen durchgeführt werden. So kann die Herstellung des Reaktionsgemischs in einem vorgelagerten (semi)-Batch Prozess durchgeführt werden wie beispielsweise in einem Rührbehälter. Das Reaktionsgemisch kann einen oder mehrere Reaktanden, bevorzugt zwei bis zehn wie beispielsweise zwei, drei, vier fünf, sechs oder mehr Reaktanden sowie gegebenenfalls Hilfsstoffe wie Lösemittel und weitere, bevorzugt homogene Katalysatoren und/oder Cokatalysatoren umfassen. Das Reaktionsgemisch wird bevorzugt in-situ erzeugt und nicht isoliert. In einer bevorzugten Ausführungsform werden die Edukte, unabhängig voneinander gegebenenfalls mit Lösemittel verdünnt, erst kurz vor dem Eintritt in das Reaktionsrohr vermischt. So hat es sich besonders bewährt, die Komponenten des Reaktionsgemischs in einer Mischstrecke zusammen zu bringen, aus der sie, gegebenenfalls nach Zwischenkühlung, in die Heizzone gefördert werden. Weiterhin bevorzugt werden die Edukte dem erfindungsgemäßen Verfahren in flüssiger Form zugeführt. Dazu können höher schmelzende und/oder höher viskose Edukte beispielsweise in geschmolzenem Zustand und/oder mit Lösemittel versetzt beispielsweise als Lösung, Dispersion oder Emulsion eingesetzt werden. Ein weiterer Katalysator kann, sofern eingesetzt, einem der Edukte oder auch der Eduktmischung vor dem Eintritt in das Reaktionsrohr zugesetzt werden. Auch gasförmige und heterogene Systeme können nach dem erfindungsgemäßen Verfahren umgesetzt werden, wobei entsprechende technische Vorrichtungen zum Fördern des Reaktionsgutes erforderlich sind. Die Teilchengröße von festen Bestandteilen des Reaktionsgemischs muss dabei deutlich unter der Porengröße des Schaums liegen. Bevorzugt beträgt die Teilchengröße fester Bestandteile, falls anwesend, höchstens 50 %, insbesondere höchstens 10 % wie beispielsweise höchstens 2 % der Porenweite des Schaums.

In einer speziellen Ausführungsform werden ein oder mehrere gasförmige Edukte eingesetzt. Beispiele für geeignete gasförmige Edukte sind Wasserstoff, Sauerstoff, Chlor, Fluor, Ethylenoxid, Propylenoxid, Kohlenmonoxid, Kohlendioxid, Schwefeldioxid, Schwefelwasserstoff, Ammoniak, Methylamin, Dimethylamin, Hydrazin und Chlorwasserstoff. Diese werden bevorzugt vor dem Eintritt des Reaktionsgemischs in das Reaktionsrohr mit einer entsprechenden Pumpe zu den übrigen Reaktanden gefördert. Bevorzugt werden beim Einsatz gasförmiger Edukte die Reaktionsbedingungen so gewählt, dass die Gase im flüssigen Zustand oder gelöst im Reaktionsgemisch vorliegen. Alternativ können die Gase auch durch Reaktion entsprechender Verbindungen in der Heizzone hergestellt werden und in-situ in die eigentliche Reaktion eingreifen. So können beispielsweise Hydrierungen entweder mit molekularem Wasserstoff oder durch Transferhydrierungen beispielsweise mit Formiaten als Wasserstoffspender durchgeführt werden. Beim Passieren des katalytisch aktiven und/oder katalytisch aktive Spezies tragenden Schaums finden dabei eine ausgezeichnete Durchmischung der Reaktanden sowie ein intensiver Kontakt der Reaktionsgemisches mit dem katalytisch aktiven und/oder katalytisch aktive Spezies tragenden Schaum statt. Auf Grund der Druckfestigkeit des Systems kann dabei auch unter hohen Drücken gearbeitet werden, ohne dass insbesondere die gasförmigen oder auch andere niedrig siedende Komponenten entweichen.

Zur Vermeidung von Nebenreaktionen und zur Herstellung von möglichst reinen Produkten hat es sich bewährt, Edukte und Produkte in Gegenwart eines inerten Schutzgases wie beispielsweise Stickstoff, Argon oder Helium zu handhaben.

Das Reaktionsgut kann in das Reaktionsrohr entweder an dem durch das Innenleiterrohr geführte Ende eingespeist werden als auch an dem entgegen gesetzten Ende.

Durch Variation von Rohrquerschnitt, Länge der Bestrahlungszone (hierunter wird die Strecke des Reaktionsrohres verstanden, in dem das Reaktionsgut Mikrowellenstrahlung ausgesetzt ist), Fließgeschwindigkeit, Geometrie des Hohlraumresonators, der eingestrahlten Mikrowellenleistung sowie der dabei erreichten Temperatur werden die Reaktionsbedingungen so eingestellt, dass die gewünschte Reaktionstemperatur schnellstmöglich erreicht wird und die Verweilzeit bei Maximaltemperatur so kurz bleibt, dass so wenig Nebenoder Folgereaktionen wie möglich auftreten. Die Regelung der für die einzelne chemische Reaktion gewünschten Reaktionsbedingungen erfolgt bevorzugt durch Steuerung der am Ende der Heizzone erreichten Temperatur des Reaktionsgemischs über die eingestrahlte Mikrowellenleistung. Der Druck wird dabei über das Entspannungsventil (Druckhaltevorrichtung) am Ende der Reaktionsstrecke so hoch eingestellt, dass das Reaktionsgemisch inklusive anfallender Produkte und Nebenprodukte nicht siedet. Bei Anwesenheit gasförmiger Reaktanden werden deren Konzentration und der Druck im Reaktionsrohr bevorzugt so eingestellt, dass die gasförmigen Reaktanden im Reaktionsgemisch gelöst oder in verflüssigter Form vorliegen.

Bevorzugt wird das Verfahren bei Drücken zwischen 1 bar (Atmosphärendruck) und 500 bar und besonders bevorzugt zwischen 1,5 und 200 bar, insbesondere zwischen 3 bar und 150 bar und speziell zwischen 10 bar und 100 bar wie beispielsweise zwischen 15 bar und 50 bar durchgeführt. Besonders bewährt hat sich das Arbeiten unter erhöhtem Druck, wobei oberhalb der Siedetemperatur (bei Normaldruck) der Edukte, Produkte, des gegebenenfalls anwesenden Lösemittels und/oder der während der Reaktion gebildeten Produkte gearbeitet wird. Besonders bevorzugt wird der Druck so hoch eingestellt, dass das Reaktionsgemisch während der Mikrowellenbestrahlung im flüssigen Zustand verbleibt und nicht siedet.

Die erfindungsgemäße Vorrichtung und das erfindungsgemäße Verfahren erlauben eine sehr schnelle, energiesparende und kostengünstige Durchführung heterogen katalysierter chemischer Reaktionen in hohen Ausbeuten in großtechnischen Mengen. Die Vorteile des erfindungsgemäßen Verfahrens liegen dabei insbesondere in einer sehr schnellen und trotzdem gezielten Erhitzung des Reaktionsgutes mittels Mikrowellen auf die angestrebte Reaktionstemperatur, ohne dass es zu wesentlichen Überschreitungen der durchschnittlichen Temperatur des Reaktionsgutes zum Beispiel an der Gefäßwand kommt. Dies ist besonders ausgeprägt bei Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators und insbesondere innerhalb eines $E_{01}$-Hohlraumresonators beispielsweise mit koaxialem Übergang der Mikrowellen befindet. Mit der erfindungsgemäßen Vorrichtung ist es im Gegensatz zu konventionellen Heiztechniken mit Wärmeübertragung mittels eines Temperaturgradienten möglich, das Reaktionsgemisch bis annähernd an die Zersetzungstemperatur der temperaturempfindlichsten Komponente zu erhitzen und bei dieser Temperatur bis zur Einstellung des für diese Bedingungen herrschenden Gleichgewichtszustand zu halten.

In der erfindungsgemäßen Vorrichtung sowie bei dem sie nutzenden Verfahren wird ein sehr hoher Wirkungsgrad bei der Ausnutzung der in den Hohlraumresonator eingestrahlten Mikrowellenenergie erzielt wird, der üblicherweise über 50 %, oftmals über 80 %, teilweise über 90 % und in speziellen Fällen über 95 % wie beispielsweise über 98 % der eingestrahlten Mikrowellenleistung liegt und somit ökonomische wie auch ökologische Vorteile gegenüber konventionellen Herstellverfahren wie auch gegenüber Mikrowellenverfahren des Standes der Technik bietet.

Erfindungsgemäße Vorrichtung und Verfahren erlauben zudem durch die kontinuierliche Mikrowellenbestrahlung nur kleiner durch das Volumen der Vorrichtung begrenzter Mengen an Reaktionsgut eine kontrollierte, sichere und reproduzierbare Reaktionsführung. Insbesondere bei Bestrahlung des Reaktionsgutes im Zentrum eines symmetrischen Mikrowellenfeldes innerhalb eines Reaktionsrohres, dessen Längsachse sich in der Ausbreitungsrichtung der Mikrowellen eines Monomode-Mikrowellenapplikators befindet, wird das Reaktionsgut während der Mikrowellenbestrahlung parallel zur Ausbreitungsrichtung der Mikrowellen bewegt. So werden bekannte Überhitzungsphänomene durch unkontrollierbare Feldverteilungen, die zu lokalen Überhitzungen durch wechselnde Intensitäten des Mikrowellenfeldes beispielsweise in Wellenbergen und Knotenpunkten führen können, durch die Fließbewegung des Reaktionsgutes ausgeglichen. Die genannten Vorteile erlauben es auch, mit hohen Mikrowellenleistungen von beispielsweise mehr als 10 kW oder mehr als 100 kW zu arbeiten und somit in Kombination mit einer nur kurzen Verweilzeit im Hohlraumresonator große Produktionsmengen von 100 und mehr Tonnen pro Jahr in einer Anlage zu bewerkstelligen.

Dabei werden verglichen mit unkatalysierten, mikrowellenunterstützten Reaktionen wie auch verglichen mit katalysierten, thermisch geheizten Reaktionen kürzere Verweilzeiten des Reaktionsguts im Reaktionsrohr benötigt, wodurch kürzere und/oder dünnere mikrowellentransparente Rohre zur Erzielung eines definierten Umsatzes benötigt werden. Dabei wurde praktisch keine Bildung thermischer Zersetzungsprodukte beobachtet, wie sie bei entsprechenden Umsetzungen in einem mit offenporigem, katalytisch aktiven oder katalytisch aktive Spezies enthaltenden Schaum gefüllten Strömungsrohr gleicher Dimensionierung unter thermischer Mantelheizung entstehen. Des Weiteren finden sich in den nach dem erfindungsgemäßen Verfahren hergestellten Produkten überraschenderweise sehr niedrige Gehalte an ausgewaschenem Katalysator, so dass es üblicherweise keiner weiteren Aufarbeitung der Rohprodukte bedarf. Durch die Verwendung von offenporigen Schäumen als Trägermaterial wird das Reaktionsgut durch die vom offenzelligen Schaum verursachte Quervermischung sehr gut durchmischt und in vielen Fällen in eine turbulente Strömung versetzt. Somit können insbesondere auch nicht oder nur begrenzt miteinander mischbare Edukte getrennt voneinander direkt vor dem Eintritt in das Reaktionsrohr dosiert werden. Somit wird das bei laminarem Fluss oftmals beobachtete nebeneinander Fließen der Edukte, ohne dass es zu einer Reaktion kommen kann, vermieden. Des Gleichen wird die Vermischung auch unvollständig miteinander mischbarer Reaktionspartner verbessert und eine Reaktion erleichtert. Zudem besitzen schaumartige Strukturen eine sehr große, katalytisch aktive Oberfläche ohne dem fließenden Reaktionsgut einen größeren Fließwiderstand entgegenzusetzen, wie es übliche Festbettkatalysatoren in granularer oder anderer fester Form zeigen. So führen die Verwendung der erfindungsgemäßen Vorrichtung und/oder des sie nutzenden Verfahrens zu erhöhten Ausbeuten an Zielprodukt und höherer Reinheit des Zielprodukts.

Beispiele

Die Bestrahlungen der Reaktionsgemische mit Mikrowellen erfolgten in einem Reaktionsrohr (60 x 1 cm) aus Aluminiumoxid, das sich axialsymmetrisch in einem zylindrischen Hohlraumresonator (60 x 10 cm) befand. Das Reaktionsrohr war über die gesamte Länge mit einem in den jeweiligen Beispielen spezifizierten offenzelligen Schaum gefüllt. An einer der Stirnseiten des Hohlraumresonators verlief das Reaktionsrohr durch den Hohlraum eines als Kopplungsantenne fungierenden Innenleiterrohres. Das von einem Magnetron erzeugte Mikrowellenfeld mit einer Frequenz von 2,45 GHz wurde mittels der Kopplungsantenne in den Hohlraumresonator eingekoppelt ($E_{01}$-Hohlraumapplikator; Monomode), in dem sich eine stehende Welle ausbildete. Nach Verlassen dieses Reaktionsrohres wurden die Reaktionsgemische, sofern nicht anders angegeben, auf Atmosphärendruck entspannt und sofort mittels eines Intensivwärmetauschers auf ca. 40 °C abgekühlt.

Die Mikrowellenleistung wurde über die Versuchsdauer jeweils in der Art eingestellt, dass die gewünschte Temperatur des Reaktionsgutes am Ende des Reaktionsrohres konstant gehalten wurde. Die in den Versuchsbeschreibungen genannten Mikrowellenleistungen repräsentieren daher den zeitlichen Mittelwert der eingestrahlten Mikrowellenleistung. Die Temperaturmessung des Reaktionsgemischs wurde direkt nach Verlassen des Reaktionsrohres (etwa 15 cm Strecke in einer isolierten Edelstahlkapillare, Ø 1 cm) mittels Pt100 Temperatursensor vorgenommen. Vom Reaktionsgemisch nicht direkt absorbierte Mikrowellenenergie wurde an der der Kopplungsantenne entgegen liegenden Stirnseite des Hohlraumresonators reflektiert; die vom Reaktionsgemisch auch beim Rücklauf nicht absorbierte und in Richtung des Magnetrons zurück gespiegelte Mikrowellenenergie wurde mit Hilfe eines Prismensystems (Zirkulator) in ein Wasser enthaltendes Gefäß geleitet. Aus der Differenz zwischen eingestrahlter Energie und Aufheizung dieser Wasserlast wurde die in das Reaktionsgut eingetragene Mikrowellenenergie berechnet

Mittels einer Hochdruckpumpe und eines Druckentlastungsventils wurde die Reaktionsmischung im Reaktionsrohr unter einen solchen Arbeitsdruck gesetzt, der ausreichte, um alle Edukte und Produkte bzw. Kondensationsprodukte stets im flüssigen Zustand zu halten. Die Reaktionsgemische wurden mit einer konstanten Flussrate durch die Vorrichtung gepumpt und die Verweilzeit im Reaktionsrohr durch Modifizierung der Strömungsgeschwindigkeit eingestellt.

Die Analytik der Produkte erfolgte mittels $^1$H-NMR-Spektroskopie bei 500 MHz in $CDCl_3$.

Beispiel 1: Herstellung von Rapsölfettsäuremethylester

In einem 10 I-Büchi-Rührautoklaven mit Rührer, Innenthermometer und Druckausgleich wurden 4,4 kg Rapsöl (5 mol, MW 878 g/mol) vorgelegt und mit 1,12 kg Methanol (35 mol) versetzt. Das so erhaltene Gemisch wurde bei einem Arbeitsdruck von 35 bar kontinuierlich mit 5 l/h durch das Reaktionsrohr gepumpt, das für diesen Versuch mit einem offenzelligen Schaum aus Cordierit mit einer Porenzahl von 50 ppi bei einer Porosität von 70 % gefüllt war. Es wurde einer Mikrowellenleistung von 2,2 kW ausgesetzt, von denen 91 % vom Reaktionsgut absorbiert wurden. Die Verweilzeit des Reaktionsgemischs im Reaktionsrohr betrug ca. 30 Sekunden. Nach Verlassen des Reaktionsrohres hatte das Reaktionsgemisch eine Temperatur von 211 °C.

Es wurde ein Umsatz des Triglycerids von 97 % d. Th. erreicht. Das Reaktionsprodukt war leicht gelblich gefärbt, sein Gehalt an Calciumionen lag unter 5 ppm (< Nachweisgrenze). Nach Abtrennung des spezifisch schwereren Glycerins und destillativer Abtrennung des überschüssigen Methanols wurden 4,2 kg Rapsölfettsäuremethylester erhalten, der als Nebenprodukte 0,5 % Diglyceride und 1,3 % Monoglyceride enthielt.

Beispiel 2: Suzuki-Kupplung

In einem mit Stickstoff inertisierten 10 I-Büchi-Rührautoklaven mit Rührer, Innenthermometer und Druckausgleich wurden 6 Liter eines Gemischs aus Ethanol, Wasser und Dimethylformamid vorgelegt und darin 1,13 kg 4-Bromtoluol (6,6 mol) und 732 g (6,0 mol) Phenylboronsäure gelöst. Anschließend wurde erneut mit Stickstoff inertisiert. Die so erhaltene Eduktlösung wurde bei einem Arbeitsdruck von 30 bar kontinuierlich mit 3,8 l/h durch das mit Palladium dotiertem Aluminiumoxidschaum gefüllte Reaktionsrohr gepumpt. Ein Aluminiumoxidschaum mit einer Porenzahl von 40 ppi und einer Porosität von 60 % war für diesen Versuch in Anlehnung an die Vorschrift von M. Organ (Angew. Chem. 2006, 118, 2827-2832) mit Pd imprägniert worden. Das Reaktionsgemisch wurde einer Mikrowellenleistung von 1,8 kW ausgesetzt, von denen 95 % vom Reaktionsgut absorbiert wurden. Die Verweilzeit des Reaktionsgemischs im Reaktionsrohr betrug ca. 39 Sekunden. Nach Verlassen des Reaktionsrohres hatte das Reaktionsgemisch eine Temperatur von 248 °C.

Es wurde eine mittels $^1$H-NMR bestimmte Ausbeute (bezogen auf die im Unterschuss eingesetzte Phenylboronsäure) von 75 % d. Th. erreicht, der Palladiumgehalt des Produktes betrug < 5 ppm (< Nachweisgrenze). Mittels Dünnschichtverdampfer wurden die verwendeten Lösungsmittel entfernt und der Produkt-Sumpf in eine Vakuumdestillationsapparatur überführt. Nach destillativer Aufreinigung (134 - 136 °C / 12 mbar) wurden 706 g 4-Methylbiphenyl mit einer Reinheit von > 99 % erhalten.

Beispiel 3: Katalytische Hydrierung von Sonnenblumenöl

In einem mit Stickstoff inertisierten 10 l Büchi-Rührautoklaven mit Rührer, Innenthermometer und Druckausgleich wurden 7 Liter Sonnenblumenöl mit einem Anteil an ungesättigten Fettsäuren von 90% vorgelegt und auf 70 °C erwärmt. Anschließend wurde das Öl bei einem Arbeitsdruck von 8 bar kontinuierlich mit 4 Liter pro Stunde durch das Reaktionsrohr gepumpt, das mit einem wie in Beispiel 2 beschriebenen Pd-dotierten Aluminiumoxidschaum gefüllt war. Dabei wurde vor Eintritt des Öls in das Reaktionsrohr Wasserstoff in den Eduktstrom in der Art eingegast, das der sich im inneren der Apparatur einstellende Druck von 8 bar nicht überschritten wurde.

Mittels einer Mikrowellenleistung von 1,7 KW wurde eine mittlere Reaktionstemperatur von 160°C am Ende des Reaktionsrohres eingestellt. Die Aufenthaltszeit des Öl/$H_2$-Gemisches im Reaktionsrohr betrug im Durchschnitt 37 Sekunden. Nach Verlassen des Reaktionsrohres wurde das Reaktionsprodukt mittels eines Wärmetauschers auf 40 °C abgekühlt.

Das resultierende gehärtete Sonnenblumenöl besaß nach erfolgter Reaktion einen Gehalt an ungesättigten Fettsäure von 7 %. Beim Stehen lassen verfestigte sich das Produkt nach kurzer Zeit zu einer weißen, wachsartigen Masse.

Beispiel 4: Katalytische Reduktion von Cyclohexanon mittels Transfer-Hydrierung zu Cyclohexanol

In einem mit Stickstoff inertisierten 10 l Büchi-Rührautoklaven mit Rührer, Innenthermometer und Druckausgleich wurden 5 Liter Isopropanol (wasserfrei) vorgelegt, unter Rühren 2,94 kg Cyclohexanon (30 mol) hinzugegeben und auf 50 °C erwärmt. Die so hergestellte Cyclohexanon-Lösung wurde anschließend bei einem Arbeitsdruck von 25 bar mit einer Flussrate von 3 Liter pro Stunde durch das mit einem mit Ruthenium imprägnierten Cordierit-Schaum mit einer Porenzahl von 30 ppi und einer Porosität von 60 % gefüllte Reaktionsrohr gepumpt.

Die eingestrahlte Mikrowellenleistung von 1,5 KW erhitzte das Reaktionsgut auf 155 °C. Die Aufenthaltszeit des Isopropanol/Cyclohexanon-Lösung im Reaktionsrohr betrug annähernd eine Minute. Nach Verlassen des Reaktionsrohres wurde das Reaktionsprodukt mittels eines Wärmetauschers auf 40 °C abgekühlt. Die nach $^1$H-NMR bestimmte Ausbeute betrug 93 % bezogen auf das eingesetzte Cyclohexanon. Der Rutheniumgehalt des Reaktionsproduktes betrug < 5ppm (< Nachweisgrenze).

Beispiel 5: Entschwefelung von Diesel

In einem 10 l-B.üchi-Rührautoklaven mit Rührer, Innenthermometer und Druckausgleich wurden 8 Liter einer Mitteldestillatfraktion ("Diesel") mit einem Siedebereich von 160 bis 365 °C und einem Schwefelgehalt von 1.000 ppm vorgelegt und mit Stickstoff inertisiert. Bei einem Arbeitsdruck von 20 bar wurde der schwefelhaltige Diesel kontinuierlich mit einer Flussrate von 5 l/h durch das Reaktionsrohr gepumpt, das für diesen Versuch mit einem offenzelligen Schaum aus einem mit Siliziumcarbid durchsetzten Aluminiumoxid mit einer Porenzahl von 75 ppi bei einer Porosität von 60 % gefüllt war, auf den eine Mischung aus Kobalt- und Molybdänoxid aufgebracht und in eine Sulfidform überführt worden war. Vor Eintritt des Diesels in das Reaktionsrohr wurde in den Dieselstrom mittels geeigneter Dosiereinrichtung $H_2$ eingepresst (1,5 Liter/h bezogen auf Normaldruck). Es wurde eine Mikrowellenleistung von 3 kW angelegt, von der 85 % vom Reaktionsgut absorbiert wurden. Die Verweilzeit des Reaktionsguts im Reaktionsrohr betrug ca. 30 Sekunden. Nach Verlassen des Reaktionsrohres hatte das Reaktionsgut eine Temperatur von 275 °C. Mittels Wärmetauscher wurde vor der Druckentlastung des Systems auf 60 °C abgekühlt und das Produkt sodann in einen Entgasungsbehälter entspannt, in dem mittels Durchleiten von Stickstoff der entstandene Schwefelwasserstoff und Reste von unreagiertem Wasserstoff abgestrippt wurden. Der so erhaltene Diesel besaß einen Restschwefelgehalt von 35 ppm.

**Patentansprüche**

1. Vorrichtung zur kontinuierlichen Durchführung heterogen katalysierter chemischer Reaktionen, umfassend ein mikrowellentransparentes Rohr, dessen Längsachse sich in der Ausbreitungsrichtung der Mikrowellen eines Monomode-Mikrowellenapplikators befindet, wobei das mikrowellentransparente Rohr mit einem offenzelligen, katalytisch aktive Spezies tragenden oder aus diesem bestehenden Schaum gefüllt ist.

2. Vorrichtung nach Anspruch 1, umfassend ein weitgehend mikrowellentransparentes Reaktionsrohr innerhalb eines mit einem Mikrowellengenerator über Wellenleiter verbundenen Hohlleiters.

3. Vorrichtung nach einem oder mehreren der Ansprüche 1 und 2, bei dem der Mikrowellenapplikator als Hohlraumresonator ausgestaltet ist.

4. Vorrichtung nach einem oder mehreren der Ansprüche 1 bis 3, bei dem der Mikrowellenapplikator als Hohlraumresonator vom Reflexionstyp ausgestaltet ist.

**5.** Vorrichtung nach einem oder mehreren der Ansprüche 1 bis 4, bei dem das Reaktionsrohr axial mit einer zentralen Symmetrieachse des Hohlleiters fluchtet.

**6.** Vorrichtung nach einem oder mehreren der Ansprüche 1 bis 5, umfassend einen Hohlraumresonator mit koaxialem Übergang der Mikrowellen.

**7.** Vorrichtung nach einem oder mehreren der Ansprüche 1 bis 6, bei dem der Hohlraumresonator so beschaffen ist, dass er im $E_{01n}$-Mode betrieben werden kann, wobei n eine ganze Zahl von 1 bis 200 ist.

**8.** Vorrichtung nach einem oder mehreren der Ansprüche 1 bis 7, bei dem der Hohlraumresonator so beschaffen ist, dass sich darin eine stehende Welle ausbilden kann.

**9.** Vorrichtung nach einem oder mehreren der Ansprüche 1 bis 8, welche die Mikrowellenbestrahlung bei Temperaturen zwischen 100 und 400 °C ermöglicht.

**10.** Vorrichtung nach einem oder mehreren der Ansprüche 1 bis 9, welche die Mikrowellenbestrahlung bei Drücken oberhalb des Atmosphärendrucks ermöglicht.

**11.** Vorrichtung nach einem oder mehreren der Ansprüche 1 - 10, worin der offenzellige Schaum das Reaktionsrohr im Bereich der Heizzone im Wesentlichen ganz füllt.

**12.** Vorrichtung nach einem oder mehreren der Ansprüche 1 - 11, worin der offenzellige Schaum aus einem katalytisch selbst aktiven keramischen Material besteht.

**13.** Vorrichtung nach einem oder mehreren der Ansprüche 1 - 11, worin der offenzellige Schaum ein keramisches Material ist, welches eine davon verschiedene katalytisch aktive Spezies trägt.

**14.** Vorrichtung nach einem oder mehreren der Ansprüche 1 - 11, worin der offenzellige Schaum aus einem katalytisch aktiven Metall besteht.

**15.** Vorrichtung nach einem oder mehreren der Ansprüche 1 - 14, worin die in Anlehnung an ASTM D3576 bestimmte Porenzahl des offenzelligen Schaums zwischen 1 und 150 ppi liegt.

**16.** Vorrichtung nach einem oder mehreren der Ansprüche 1 - 15, worin die Porosität des offenzelligen Schaums mindestens 20 % beträgt.

**17.** Vorrichtung nach einem oder mehreren der Ansprüche 1 - 16, worin die Porenweite des offenzelligen Schaums zwischen 0,01 mm und 1 cm liegt.

**18.** Vorrichtung nach einem oder mehreren der Ansprüche 1 - 17, worin die über Stempeleindruck bestimmbare durchschnittliche Festigkeit des offenzelligen Schaums zwischen 100 und 15.000 N beträgt.

**19.** Vorrichtung nach einem oder mehreren der Ansprüche 1 - 18, worin das mit einem offenzelligen, katalytisch aktive Spezies tragenden oder aus diesem bestehenden Schaum gefüllte, mikrowellentransparente Rohr so ausgeführt ist, dass der vom offenzelligen Schaum bedingte Druckverlust weniger als $5 \cdot 10^5$ Pa/m beträgt.

**20.** Verfahren zur kontinuierlichen Durchführung heterogen katalysierter chemischer Reaktionen, bei dem das Reaktionsgut durch Bestrahlung mit Mikrowellen in einem mikrowellentransparenten Rohr, dessen Längsachse sich in der Ausbreitungsrichtung der Mikrowellen eines Monomode-Mikrowellenapplikators befindet, umgesetzt wird, wobei das mikrowellentransparente Rohr mit einem offenzelligen, katalytisch aktive Spezies tragenden oder aus diesem bestehenden Schaum gefüllt ist.

**21.** Verfahren gemäß Anspruch 20, wobei die heterogen katalysierten Reaktionen in einer Vorrichtung gemäß einem oder mehreren der Ansprüche 1 bis 19 durchgeführt werden.

**Claims**

**1.** An apparatus for continuous performance of heterogeneously catalyzed chemical reactions, comprising a microwave-transparent tube whose longitudinal axis is in the direction of propagation of the microwaves from a monomode microwave applicator, the microwave-transparent tube being filled with an open-cell foam bearing or consisting of catalytically active species.

**2.** The apparatus as claimed in claim 1, comprising a substantially microwave-transparent reaction tube within a hollow conductor connected via waveguides to a microwave generator.

**3.** The apparatus as claimed in one or more of claims 1 and 2, in which the microwave applicator is configured as a cavity resonator.

**4.** The apparatus as claimed in one or more of claims 1 to 3, in which the microwave applicator is configured as a cavity resonator of the reflection type.

**5.** The apparatus as claimed in one or more of claims 1 to 4, in which the reaction tube is aligned axially with a central axis of symmetry of the hollow conductor.

**6.** The apparatus as claimed in one or more of claims 1 to 5, comprising a cavity resonator with a coaxial transition of the microwaves.

**7.** The apparatus as claimed in one or more of claims 1 to 6, in which the cavity resonator is configured such that it can be operated in $E_{01n}$ mode where n is an integer from 1 to 200.

**8.** The apparatus as claimed in one or more of claims 1 to 7, in which the cavity resonator is configured such that a standing wave can form therein.

**9.** The apparatus as claimed in one or more of claims 1 to 8, which enables microwave irradiation at temperatures between 100 and 400°C.

**10.** The apparatus as claimed in one or more of claims 1 to 9, which enables microwave irradiation at pressures above atmospheric pressure.

**11.** The apparatus as claimed in one or more of claims 1 - 10, in which the open-cell foam essentially fills the reaction tube in the region of the heating zone.

**12.** The apparatus as claimed in one or more of claims 1 - 11, in which the open-cell foam consists of a ceramic material which is itself catalytically active.

**13.** The apparatus as claimed in one or more of claims 1 - 11, in which the open-cell foam is a ceramic material which bears a different catalytically active species.

**14.** The apparatus as claimed in one or more of claims 1 - 11, in which the open-cell foam consists of a catalytically active metal.

**15.** The apparatus as claimed in one or more of claims 1 - 14, in which the pore count of the open-cell foam, determined on the basis of ASTM D3576, is between 1 and 150 ppi.

**16.** The apparatus as claimed in one or more of claims 1 - 15, in which the porosity of the open-cell foam is at least 20%.

**17.** The apparatus as claimed in one or more of claims 1 - 16, in which the pore size of the open-cell foam is between 0.01 mm and 1 cm.

**18.** The apparatus as claimed in one or more of claims 1 - 17, in which the average strength of the open-cell foam, determinable by means of die indentation, is between 100 and 15 000 N.

**19.** The apparatus as claimed in one or more of claims 1 - 18, in which the microwave-transparent tube filled with an open-cell foam bearing or consisting of catalytically active species is configured such that the pressure drop caused by the open-cell foam is less than $5.10^5$ Pa/m.

**20.** A process for continuously performing heterogeneously catalyzed chemical reactions, in which the reaction mixture is converted by irradiation with microwaves in a microwave-transparent tube whose longitudinal axis is in the direction of propagation of the microwaves from a monomode microwave applicator, the microwave-transparent tube being filled with an open-cell foam bearing or consisting of catalytically active species.

**21.** The process as claimed in claim 20, wherein the heterogeneously catalyzed reactions are performed in an apparatus as claimed in one or more of claims 1 to 19.

**Revendications**

**1.** Dispositif pour la réalisation continue de réactions chimiques sous catalyse hétérogène, comprenant un tube transparent aux micro-ondes, dont l'axe longitudinal se trouve dans la direction de propagation des micro-ondes d'un applicateur de micro-ondes monomodal, le tube transparent aux micro-ondes étant rempli avec une mousse à cellules ouvertes, portant une espèce catalytiquement active ou constituée de celle-ci.

**2.** Dispositif selon la revendication 1, comprenant un tube de réaction essentiellement transparent aux micro-ondes dans un conducteur creux raccordé avec un générateur de micro-ondes par un guide d'ondes.

**3.** Dispositif selon une ou plusieurs des revendications 1 et 2, dans lequel l'applicateur de micro-ondes est configuré sous la forme d'une cavité résonante.

**4.** Dispositif selon une ou plusieurs des revendications 1 à 3, dans lequel l'applicateur de micro-ondes est configuré sous la forme d'une cavité résonante de type à réflexion.

**5.** Dispositif selon une ou plusieurs des revendications 1 à 4, dans lequel le tube de réaction est aligné axialement avec un axe de symétrie central du conducteur creux.

**6.** Dispositif selon une ou plusieurs des revendications 1 à 5, comprenant une cavité résonante qui présente un croisement coaxial des micro-ondes.

**7.** Dispositif selon une ou plusieurs des revendications 1 à 6, dans lequel la cavité résonante est conçue de sorte qu'elle puisse être utilisée en mode $E_{01n}$, n étant un nombre entier de 1 à 200.

8. Dispositif selon une ou plusieurs des revendications 1 à 7, dans lequel la cavité résonante est conçue de sorte qu'une onde stationnaire puisse se former dans celle-ci.

9. Dispositif selon une ou plusieurs des revendications 1 à 8, qui permet l'exposition à des micro-ondes à des températures comprises entre 100 et 400 °C.

10. Dispositif selon une ou plusieurs des revendications 1 à 9, qui permet l'exposition à des micro-ondes à des pressions supérieures à la pression atmosphérique.

11. Dispositif selon une ou plusieurs des revendications 1 à 10, dans lequel la mousse à cellules ouvertes remplit essentiellement en totalité le tube de réaction dans la partie de la zone de chauffage.

12. Dispositif selon une ou plusieurs des revendications 1 à 11, dans lequel la mousse à cellules ouvertes est constituée d'un matériau céramique catalytiquement auto-actif.

13. Dispositif selon une ou plusieurs des revendications 1 à 11, dans lequel la mousse à cellules ouvertes est un matériau céramique qui porte une espèce catalytiquement active différente de celui-ci.

14. Dispositif selon une ou plusieurs des revendications 1 à 11, dans lequel la mousse à cellules ouvertes est constituée d'un métal catalytiquement actif.

15. Dispositif selon une ou plusieurs des revendications 1 à 14, dans lequel le nombre de pores de la mousse à cellules ouvertes, déterminé selon ASTM D3576, est compris entre 1 et 150 ppi.

16. Dispositif selon une ou plusieurs des revendications 1 à 15, dans lequel la porosité de la mousse à cellules ouvertes est d'au moins 20 %.

17. Dispositif selon une ou plusieurs des revendications 1 à 16, dans lequel la largeur de pore de la mousse à cellules ouvertes est comprise entre 0,01 mm et 1 cm.

18. Dispositif selon une ou plusieurs des revendications 1 à 17, dans lequel la résistance moyenne de la mousse à cellules ouvertes déterminable par l'effet d'un piston est comprise entre 100 et 15 000 N.

19. Dispositif selon une ou plusieurs des revendications 1 à 18, dans lequel le tube transparent aux micro-ondes rempli avec une mousse à cellules ouvertes portant une espèce catalytiquement active ou constitué de celle-ci est configuré de sorte que la perte de charge causée par la mousse à cellules ouvertes soit inférieure à $5 \cdot 10^5$ Pa/m.

20. Procédé de réalisation continue de réactions chimiques sous catalyse hétérogène, selon lequel le mélange réactionnel est mis en réaction par exposition à des micro-ondes dans un tube transparent aux micro-ondes, dont l'axe longitudinal se trouve dans la direction de propagation des micro-ondes d'un applicateur de micro-ondes monomodal, le tube transparent aux micro-ondes étant rempli avec une mousse à cellules ouvertes, portant une espèce catalytiquement active ou constituée de celles-ci.

21. Procédé selon la revendication 20, dans lequel les réactions sous catalyse hétérogène sont réalisées dans un dispositif selon une ou plusieurs des revendications 1 à 19.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009064501 A
- WO 2006024167 A
- CN 1351954


**In der Beschreibung aufgeführte Nicht-Patentliteratur**

- **A. LOUPY.** Microwaves in Organic Synthesis. Wiley, 2006
- **WOLF et al.** *AOSTRA J. of Research,* 1986, vol. 3, 53
- **SHORE et al.** *Angew. Chem.,* 2006, vol. 118, 2827-2832
- **MAZZOCCHIA et al.** *C. R. Chemie,* 2004, vol. 7, 601-605
- **ESVELD et al.** *Chem. Eng. Technol.,* 2000, vol. 23, 429-435
- **K. LANGE ; K.H. LÖCHERER.** *Taschenbuch der Hochfrequenztechnik,* vol. 2, K21 ff
- **D. BOGDAL.** Microwave-assisted Organic Synthesis. Elsevier, 2005
- **M. ORGAN.** *Angew. Chem.,* 2006, vol. 118, 2827-2832